# EUROPEAN PATENT APPLICATION

(11) **EP 1 419 770 A1**
(43) Date of publication of application: **19.05.2004**
(21) Application number: 01958448.1
(22) Date of filing: 24.08.2001
(51) Int. Cl.: A61K 31/415, A61K 31/427, A61K 31/42, A61K 31/421, A61K 31/454, A61K 31/506, A61K 31/40, A61K 31/4025, A61K 31/381, A61K 31/4436, A61K 31/426, A61K 31/41, A61K 31/433, A61K 31/422, A61K 31/4439, A61K 31/501, A61K 31/5377, A61K 31/351, A61K 31/4164, A61K 31/4178

(54) **APO AI EXPRESSION ACCELERATING AGENT**

(71) Applicant: SHIONOGI & CO., LTD., Osaka-shi, Osaka 541-0045 (JP)
(72) Inventor: ISHIZUKA, Natsuki, c/o Shionogi & Co., Ltd., Osaka-shi, Osaka 553-0002 (JP); NAGATA, Kiyoshi, c/o Shionogi & Co., Ltd., Osaka-shi, Osaka 553-0002 (JP); YAMAMORI, Teruo, HYOGO 665-0015 (JP); SAKAI, Katsunori, c/o Shionogi & Co., Ltd., Osaka 561-0825 (JP)
(74) Representative: Hayes, Adrian Chetwynd
(86) International application number: PCT/JP2001/007238
(87) International publication number: WO 2003/018008

(57) **Abstract**

Pharmaceutical compositions for enhancing the expression of apoAI are provided.

Pharmaceutical compositions for enhancing the expression of apoAI which comprises a compound of formula (I): 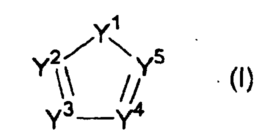 in which Y¹ is O, S or NR¹; Y², Y³, Y⁴ and Y⁵ are CR² or N; CR³ or N; CR⁴ or N; and CR⁵ or N, respectively; R¹ is A¹, -Z-A², a hydrogen, a lower alkyl and the like; R², R³, R⁴ and R⁵ are A¹, -Z-A², a hydrogen, a halogen, and like; -Z- is a single bond, -CR⁶=CR⁷-, and the like; R⁶ and R⁷ are a hydrogen or a lower alkyl; and A¹ and A² are an aryl, a heterocyclic ring, and the like; are disclosed.

## Description

### FILED OF THE INVENTION

This invention relates to a pharmaceutical composition for preventing and/or treating arteriosclerotic diseases or blood lipid disorders, and specifically to a pharmaceutical composition for enhancing the expression of apoAI.

### BACKGROUND ART

Cholesterol is well known as a main etiologic factor for arteriosclerosis that causes severe heart diseases. Especially, increased levels of serum low density lipoprotein (LDL) are believed to be a definite risk factor for coronary heart diseases (CHDs). Remedies for decreasing the level of LDL-cholesterol (LDL-C) in plasma by use of statins have been shown to be clinically effective in preventing the onset of CHDs and improving the conditions of CHDs and survivals in patients suffering from hypercholesterolemia. However, about 40 % of CHDs patients have a normal level of LDL-C, and are not always cured effectively by remedies for decreasing the level of LDL-C. On the other hand, it has been known that a half of CHDs patients having a normal level of LDL-C shows a lower level of high density lipoprotein (HDL) cholesterol (HDL-C).

Epidemiological trials in Europe and the U.S. such as Framingham studies and MRFIT (Multiple Risk Factor Intervention Trial) have reported that incidence of coronary heart diseases is higher when the level of HDL-C is lower. Other reports show that patients having only a lower level of HDL-C with normal levels of total cholesterol and triglyceride increased in a risk of arteriosclerosis. Those suggest that a low level of serum HDL-C (less than 35 to 40mg/dl) should be an independent risk factor of CHD, and the risk of complications in coronary artery diseases rapidly increases.

HDL plays an important role in reverse cholesterol transport system that is known as a biological mechanism to transfer an excess cholesterol in cells back to liver so as to maintain the level of cholesterol in living bodies normally.

Lipoproteins such as HDL is mainly comprised of lipids and proteins called apoprotein, and HDL comprises an apoprotein as referred to apolipoprotein AI (hereinafter, made up by apoAI) as a main component.

Excess free cholesterols (FCs) and phospholipids in peripheral cells are extracted by free apoAI to form lipoproteins called preβ-HDL(s). The excess FCs integrated in the preβ-HDLs are transformed into cholesteryl esters (CEs) by lecithin : cholesterol acyl transferase (LCAT), while the preβ-HDLs increase in their particle size to mature into spherical HDLs (HDL3s). The matured HDLs are classified into diverse subfractions based on the density, and these particles further grow up them to form HDL2(s). CEs are continuously transferred into VLD and LDL by means of cholesteryl ester-transporter protein (CETP). Those lipoproteins that integrate CEs are finally taken into the liver via receptors. During the course, apoAI is regenerated, and again interacts with peripheral cells to repeat the extracting of cholesterols and the regeneration of preβ-HDLs.

It has been well understood that HDL plays a central role in reverse cholesterol transport system and is a defensive factor of arteriosclerosis. It is expected that agents that promote the HDL functions would be clinically effective as medicaments for treating arteriosclerotic diseases. Accordingly, researches and developments of screening for agents that enhance in the level of HDL in plasma have been conducted via various approaches.

Among the possible approaches, one of the most likely effective approaches is to enhance the serum level of apoAI, a main component of HDL. Although increased level of HDL does not necessarily correlate with the level of apoAI, it is apparent in view of the role of apoAI in reverse cholesterol transport system that the increased level of apoAI is directly responsible for the promotion of the HDL functions. Actually, it has been shown that the mRNA level of apoAI in liver correlates closely to the levels of apoAI protein and HDL in blood (Dueland S, France D, Wang SL, Trawick JD, and Davis RA, J. Lipid Res. 38:1445-53 (1997), "Cholesterol 7alpha-hydroxylase influences the expression of hepatic apoA-I in two inbred mouse strains displaying different susceptibilities to atherosclerosis and in hepatoma cells."). Accordingly, it would be believed that the increase in the expression level of apoAI gene could elevate the serum level of apoAl, and consequently improve the HDL functions, leading to the activation of reverse cholesterol transport system. Actually, it has been shown that apoAI-transgenic mice and rabbit pathologic models administered with apoAI exhibit anti-arteriosclerosis activities (Rubin E.M., Krauss R.M., Spangler E. A., Verstuyft J. G., and Clift S. M., Nature 353, 265-267 (1991), "Inhibition of early atherogenesis in transgenic mice by human apolipoprotein AI."; Plump A.S., Scott C.J., Breslow J.L., Proc. Natl. Acad. Sci. USA., 91, 9607-9611 (1994), "Human apolipoprotein A-I gene expression increases high density lipoprotein and suppress atherosclerosis in the apolipoprotein E-deficient mouse. "; Miyazaki A., Sakuma S., Morikawa W., Takiue T., Miake F., Terano T., Sakai M., Hakamata H., Sakamoto Y., et al., Arterioscler. Thromb. Vasc. Biol. 15, 1882-1888 (1995) "Intravenous injection of rabbit apolipoprotein A-I inhibits the progression of atherosclerosis in cholesterol-fed rabbits.").

Taking into account those facts, the inventors of the present application believe that agents that activate apoAI would be candidates for medicaments of blood lipid disorders, arteriosclerotic diseases, and other diverse diseases involving HDL.

Compounds that elevate HDL are described in W097/19931, WO97/19932, US5599829, and EP796874, whereas compounds that increase apoAI are described in Japanese Patent Publication (kokai) No. 221959/1993, Japanese Patent Publication (kokai) No. 291094/1996, and W097/09048. However, those compounds are different from the compounds according to the present invention in terms of their chemical structure.

### DISCLOSURE OF THE INVENTION

The present invention is directed to pharmaceutical compositions for enhancing excellently the expression of apoAI.

Specifically, the invention provides
1) A pharmaceutical composition for enhancing the expression of apoAI, which comprises a compound of formula (I): 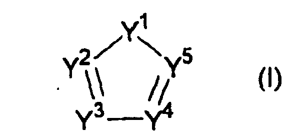 in which
   Y¹ is O, S or NR¹;
   Y² is CR² or N;
   Y³ is CR³ or N;
   Y⁴ is CR⁴ or N;
   Y⁵ is CR⁵ or N;
   R¹ is A¹, -Z-A², a hydrogen, a lower alkyl that may be optionally substituted, an acyl that may be optionally substituted, an amino that may be optionally substituted, a lower alkoxycarbonyl that may be optionally substituted, or a carbamoyl that may be optionally substituted;
   R², R³, R⁴ and R⁵ are independently A¹, -Z-A², a hydrogen, a halogen, a hydroxy, a lower alkyl that may be optionally substituted, a lower alkoxy that may be optionally substituted, a nitro, an acyl that may be optionally substituted, an amino that may be optionally substituted, a mercapto, a lower alkylthio that may be optionally substituted, a carboxy, a lower alkoxycarbonyl that may be optionally substituted, or a carbamoyl that may be optionally substituted;
   A¹ and A² are independently a cycloalkyl that may be optionally substituted, an aryl that may be optionally substituted, or a heterocyclic ring that may be optionally substituted;
   -Z- is a single bond, -CR⁶=CR⁷-, or -N-, wherein R⁶ and R⁷ are independently a hydrogen or a lower alkyl;
   provided that at least one selected from Y¹, Y², Y³, Y⁴, and Y⁵ has A¹, and any one of the others has -Z-A²; a prodrug thereof, a pharmaceutically acceptable salt or solvate of them;
2) The pharmaceutical composition according to above 1), in which the 5-membered ring consisting of Y¹, Y², Y³, Y⁴, and Y⁵ has a nucleus selected from a group consisting of 1,2,3-triazole, 1,2,4-triazole, 1,2,3-thiadiazole, 1,2,4-thiadiazole, 1,2,5-thiadiazole, 1,3,4-thiadiazole, 1,2,3-oxadiazole, 1,2,4-oxadiazole, 1,2,5-oxadiazole, 1,3,4-oxadiazole, pyrazole, tetrazole, oxazole, isoxazole, thiazole, isothiazole, pyrrole, furan, and thiophene;
3) The pharmaceutical composition according to above (2), in which the 5-membered ring consisting of Y¹, Y², Y³, Y⁴, and Y⁵ has a nucleus selected from a group consisting of 1,2,3-triazole, 1,2,4-triazole, 1,2,4-thiadiazole, 1,3,4-thiadiazole, 1,2,4-oxadiazole, 1,3,4-oxadiazole, pyrazole, tetrazole, oxazole, isoxazole, thiazole, furan, and thiophene;
4) The pharmaceutical composition according to any one of above (1) to (3), in which A¹ and A² are independently a phenyl, a pyridyl, a pyrazinyl, a furyl, a thienyl, a thiazolyl, a pyrazolyl, a isoxazolyl, a benzofuryl, or an indolyl, each of which may be optionally substituted;
5) The pharmaceutical composition according to above (4), in which A¹ and A² are independently a phenyl that may be optionally substituted by a halogen, a hydroxy, a lower alkyl, a lower alkoxy, a lower alkylthio, an amino that may be optionally substituted by a lower alkyl, a phenyl, a styryl or a heteroaryl; a thiazolyl that may be optionally substituted by a lower alkyl; a pyrazolyl that may be optionally substituted by a lower alkyl; an unsubstituted pyridyl; an unsubstituted indolyl; an unsubstituted benzofuryl; an unsubstituted thienyl; or an unsubstituted furyl;
6) The pharmaceutical composition according to any one of above (1) to (5), in which Z is a single bond;
7) The pharmaceutical composition according to any one of above (1) to (6), in which Y¹ is O, S or NR¹, R¹ is a lower alkyl that may be optionally substituted, or an amino that may be optionally substituted; and, among Y², Y³, Y⁴ and Y⁵, one or two is (are) independently CA¹, one is CA², and the others are independently CH or N;
8) The pharmaceutical composition according to any one of above (1) to (7), which is used for prevention and/or treatment of blood lipid disorders, arteriosclerotic diseases or coronary artery diseases;
9) A method of enhancing the expression of apoAI, which comprises administrating a therapeutically effective amount of a compound of formula (I) as defined in above (1), a prodrug thereof, a pharmaceutically acceptable salt or solvate of them to a patient expected to enhance the expression of apoAI; preferably, the method which comprises administrating a therapeutically effective amount of a compound of formula (I) as defined in above (2) to (8), a prodrug thereof, a pharmaceutically acceptable salt or solvate of them;
10) A method of treatment and/or prevention of blood lipid disorders, arteriosclerotic diseases or coronary artery diseases, which comprises administrating a therapeutically effective amount of a compound of formula (I) as defined in above (1), a prodrug thereof, a pharmaceutically acceptable salt or solvate of them to a patient suspected to have blood lipid disorders, arteriosclerotic diseases or coronary artery diseases; preferably, the method which comprises administrating a therapeutically effective amount of a compound of formula (I) as defined in above (2) to (8), a prodrug thereof, a pharmaceutically acceptable salt or solvate of them;
11) Use of a compound of formula (I) as defined in above (1), a prodrug thereof, a pharmaceutically acceptable salt or solvate of them for the manufacturing a medicament of enhancing the expression of apoAI; preferably, the use of a compound of formula (I) as defined in above (2) to (8), a prodrug thereof, a pharmaceutically acceptable salt or solvate of them;
12) Use of a compound of formula (I) as defined in above (1), a prodrug thereof, a pharmaceutically acceptable salt or solvate of them for the manufacturing a medicament of treatment and/or prevention of blood lipid disorders, arteriosclerotic diseases or coronary artery diseases; preferably, the use of a compound of formula (I) as defined in above (2) to (8), a prodrug thereof, a pharmaceutically acceptable salt or solvate of them.

When a compound according to the invention has two or more substituents: A¹, then they may be the same or different each other.

The term "halogen" as used herein includes fluorine, chlorine, bromine and iodine.

The term "lower alkyl" as used herein refers to a straight or branched chain alkyl comprising 1 to 6 carbon atoms, preferably 1 to 3 carbon atoms. Examples of the lower alkyl include methyl, ethyl, n-propyl, isopropyl, n-butyl, isobutyl, sec-butyl, tert-butyl, n-pentyl, isopentyl, neopentyl, hexyl, isohexyl, and the like.

The term "lower alkyl that may be optionally substituted" as used herein includes a lower alkyl, of which any position may be substituted by one or more substituents. The substituent may be a halogen, a hydroxy, a lower alkoxy, an aryl, an acyl, an acyloxy, a carboxy, a lower alkoxycarbonyl, an amino, a lower alkylamino, a nitro, a heteroaryl, and the like.

Alkyl moiety of "lower alkoxy", "lower alkylthio" or "lower alkylamino" is similar to the "lower alkyl" as described above.

Substituent in "lower alkoxy that may be optionally substituted" and "lower alkylthio that may be optionally substituted" is similar to the substituent of "lower alkyl that may be optionally substituted" as described above.

The term "lower alkylenedioxy" specifically includes methylenedioxy and ethylenedioxy.

Lower alkyl moiety of "lower alkoxycarbonyl" is similar to the "lower alkyl" as described above, and substituent of "lower alkoxycarbonyl that may be optionally substituted" is similar to the substituent of "lower alkyl that may be optionally substituted" as described above.

The term "acyl" as used herein includes an aroyl and an aliphatic acyl containing 1 to 7 carbon atoms. Here, "aroyl" refers to a group wherein an aryl or a heteroaryl group is bound to a carbonyl group. Examples of the acyl are formyl, acetyl, propionyl, butyryl, isobutyryl, valery, pivaloyl, hexanoyl, acryloyl, propiolyl, methacryloyl, crotonoyl, benzoyl and the like. Preferably, acetyl and benzoyl are exemplified.

Substituent of "acyl that may be optionally substituted" is similar to the substituent of the "lower alkyl that may be optionally substituted" as described above. Aroyl may be substituted by a lower alkyl. Acyl may be substituted at one or more positions by such a substituent.

Acyl moiety of "acyloxy" is similar to the "acyl" as described above.

The term "amino that may be optionally substituted" as used herein refers to an unsubstituted, mono-substituted, or di-substituted amino. Examples of the substituents include the substituents of the "lower alkyl that may be optionally substituted" as described above, and a lower alkyl. Preferably, an unsubstituted amino, a lower alkylamino, a di-lower alkylamino, a benzylamino, and an acylamino are exemplified.

Substituent of "carbamonyl that may be optionally substituted" is similar to the substituent of the "lower alkyl that may be optionally substituted" as described above. Preferably, an unsubstituted carbamoyl and a di-lower alkylcarbamoyl are exemplified.

The term "cycloalkyl" as used herein refers to an aliphatic cyclic carbon ring group containing 3 to 10 carbon atoms, preferably 3 to 6 carbon atoms. This includes cyclopropyl, cyclobutyl, cyclopentyl, cyclohexyl, cycloheptyl, cyclooctyl, cyclononyl, cyclodecyl, and the like.

Substituent of "cycloalkyl that may be optionally substituted" is similar to the substituent of the "lower alkyl that may be optionally substituted" as described above.

The term "aryl" as used herein includes, for example, phenyl, naphthyl, indanyl, indenyl, and anthryl. Phenyl and naphthyl are preferable, and phenyl is most preferable.

The term "heteroaryl" as used herein refers to a monocyclic and bicyclic aromatic heterocyclic ring group containing one or more hetero atoms selected from the group consisting of N, S and O within its ring. Examples of the heteroaryl include a monocyclic group, e.g., pyrrolyl, imidazolyl, pyrazolyl, pyridyl, pyridazinyl, pyrimidyl, pyrazinyl, triazolyl, triazinyl, tetrazolyl, isoxazolyl, oxazolyl, oxadiazolyl, isothiazolyl, thiazolyl, thiadiazolyl, furyl, thienyl, and the like; as well as a bicyclic ring group, e.g., indolyl, isoindolyl, indolizinyl, benzimidazolyl, indazolyl, cinnolinyl, phthalazinyl, benzoxazolyl, benzisoxazolyl, benzoxadiazolyl, benzothiazolyl, benzisothiazolyl, benzothiadiazolyl, benzofuryl, isobenzofuryl, benzothienyl, benzotriazolyl, imidazopyridyl, triazolopyridyl, imidazothiazolyl, pyrazinopiridazinyl, quinazolinyl, quinolinyl, isoquinolinyl, quinoxalinyl, purinyl, pteridinyl, naphthylidinyl, pyrazinopyridazinyl, and the like. Preferably, pyrrolyl, imidazolyl, pyrazolyl, pyridyl, pyrimidyl, pyrazinyl, triazolyl, tetrazolyl, isoxazolyl, oxazolyl, oxadiazolyl, isothiazolyl, thiazolyl, thiadiazolyl, furyl, thienyl, indolyl, benzoxazolyl, benzofuryl, benzothienyl, and the like.

The term "heterocyclic ring" refers to the "heteroaryl" as described above, as well as a monocyclic or bicyclic non-aromatic ring group containing one or more hetero atoms selected from the group consisting of N, S and O within its ring. Examples of the non-aromatic heterocyclic ring include a monocyclic group dioxanyl, dioxazinyl, dioxolanyl, dioxolyl, dithiazinyl, imidazolidinyl, imidazolinyl, morpholyl, morpholino, oxazinyl, oxadiazyl, furazanyl, oxathianyl, oxathiazinyl, oxathiolanyl, oxazolidinyl, oxazolinyl, piperazinyl, piperidinyl, pyranyl, pyrazolidinyl, pyrazolinyl, pyrrolidinyl, pyrrolinyl, tetrahydropyranyl, thiadiazolidinyl, thianyl, thiazinyl, thiadiazinyl, thiiranyl, thioranyl, and the like; as well as a bicyclic group chromanyl, 2H-chromenyl, coumarinyl, coumaranonyl, 1,3-dioxaindanyl, indolinyl, isoindolinyl, dihydroquinolyl, dihydroisoquinolyl, tetrahydroquinolyl, tetrahydroisoquinolyl, 6,7-dihydro-5H-[1]-pyrimidinyl, benzothiazinyl, tetrahydroquinoxalyl, cyclo-pentenopyridinyl, 4,5,6,7-tetrahydro-1H-indolyl, 4-oxochromenyl, 3,4-dihydro-2H-benzo[1,4]oxazinyl and pyrrolidinyl, and the like.

Substituents of "aryl that may be optionally substituted" and "heterocyclic ring that may be optionally substituted" in A¹ and A² include a halogen; a hydroxy; a lower alkyl optionally substituted by a halogen, a hydroxy or a lower alkoxy; a lower alkoxy optionally substituted by a halogen, a hydroxy, a carboxy or a lower alkoxycarbonyl; a lower alkenyl optionally substituted by a halogen, a hydroxy, a carboxy, a lower alkoxycarbonyl or a phenyl; a lower alkenyloxy optionally substituted by a halogen or a hydroxy; a mercapto; a lower alkylthio; a cycloalkyl optionally substituted by a halogen, a hydroxy or a lower alkyl; an acyl optionally substituted by a lower alkyl; an acyloxy; a carboxy; a lower alkoxycarbonyl; a lower alkenyloxycarbonyl; an amino optionally substituted by a lower alkyl or an acyl; a hydrazino; a carbamoyl optionally substituted by a lower alkyl; a lower alkylsulfonyl; a nitro; a cyano; an aryl optionally substituted by a halogen, a hydroxy, a lower alkyl or a lower alkoxy; a heterocyclic ring; a phenoxy optionally substituted by a halogen, a hydroxy or a lower alkyl; a monocyclic heteroaryloxy; a phenylamino optionally substituted by a halogen, a hydroxy or a lower alkyl; an oxo; and a lower alkylenedioxy; and the like. Such the substituents may be bound at one or more arbitrary positions.

The compounds according to the invention include pharmaceutically acceptable, producible salts. Examples of the "pharmaceutically acceptable salts" include a salt with an inorganic acid e.g. those with hydrochloric acid, sulfuric acid, nitric acid, phosphoric acid, or the like; a salt with an organic acid e.g. those with p-toluenesulfonic acid, methanesulfonic acid, oxalic acid, citric acid, or the like; a salt with an organic base e.g. ammonium, trimethylammonium, triethylammonium, or the like; a salt with an alkaline metal e.g. sodium or potassium, or the like; a quaternary salt with alkyl halide e.g., methyl iodide, ethyl iodide or the like; and a salt with an alkaline earth metal e.g., calcium or magnesium, or the like.

The compounds according to the invention may form solvates as coordinated with a suitable organic solvent and/or water. Hydrates are preferable.

The compounds according to the invention also include prodrugs. In the context of the invention, a "prodrug" is a derivative of a compound according to the invention comprising a chemically or metabolically cleavable group. In the course of metabolism in the body, a prodrug shows a pharmacological activity as a result of conversion to the compounds according to the invention. Method for selecting and producing suitable prodrug derivatives are described in, e.g. "Design of Prodrugs, Elsevier, Amsterdam (1985)".

Prodrugs of a compound according to the invention having a carboxy are exemplified by an ester derivative produced by condensing the carboxy group with a suitable alcohol, e.g., COORA wherein RA is a lower alkyl, a lower alkenyl or an aryl, each of which may be optionally substituted in which the substituent may be a hydroxy, an acyloxy, a carboxy, a sulfonic acid, an amino, a lower alkylamino, or the like; and alternatively by an amide derivative produced by reacting the carboxy and a suitable amine, e.g., CONRBRC wherein RB is a hydrogen, a lower alkyl, or the like; and RC is a hydrogen, a lower alkyl, an amino, a hydroxy, or the like.

Prodrugs of a compound according to the invention having a hydroxy are exemplified by an acyloxy derivative produced by reacting the hydroxy group and a suitable acyl halide or a suitable acid anhydride, e.g., -OCORA wherein RA is as defined above.

Prodrugs of a compound according to the invention having an amino are exemplified by an amide derivative produced by reacting the amino group and a suitable acid halide or a suitable mixed anhydride compound, e.g., NHCORA, and NHCOORA wherein RA is as defined above.

When compound (I) according to the invention has an asymmetric carbon atom, then the invention encompasses a racemic mixture, both of enantiomers, and all of diastereomers. When compound (I) according to the invention has a double bond, the invention may include both of geometric isomers resulting from possible arrangements of its substituents.

Although all of the compounds according to the invention have an activity for enhancing the expression of apoAI, the following compounds that comprise one group: A¹ and one group: A² can be listed as preferable compounds.

### Compounds of formula (I):

(1) wherein the 5-membered ring consisting of Y¹, Y², Y³, Y⁴ and Y⁵ is:
   1,2,3-triazole having one of A¹ and A² at position 1 and the other at position 4 (hereinafter Y-1);
   1,2,4-oxadiazole having one of A¹ and A² at position 3 and the other at position 5 (hereinafter Y-2);
   1,2,4-triazole having one of A¹ and A² at position 3 and the other at position 5 (hereinafter Y-3);
   1,3,4-oxadiazole having one of A¹ and A² at position 2 and the other at position 5 (hereinafter Y-4);
   1,2,4-thiadiazole having one of A¹ and A² at position 3 and the other at position 5 (hereinafter Y-5);
   1,3,4- thiadiazole having one of A¹ and A² at position 2 and the other at position 5 (hereinafter Y-6);
   furan having one of A¹ and A² at position 2 and the other at position 5 (hereinafter Y-7);
   isoxazole having one of A¹ and A² at position 3 and the other at position 5 (hereinafter Y-8);
   oxazole having one of A¹ and A² at position 2 and the other at position 4 (hereinafter Y-9);
   oxazole having one of A¹ and A² at position 2 and the other at position 5 (hereinafter Y-10);
   pyrazole having one of A¹ and A² at position 3 and the other at position 5 (hereinafter Y-11);
   tetrazole having one of A¹ and A² at position 2 and the other at position 5 (hereinafter Y-12);
   thiazole having one of A¹ and A² at position 2 and the other at position 4 (hereinafter Y-13);
   thiazole having one of A¹ and A² at position 2 and the other at position 5 (hereinafter Y-14); or
   1,2,4-triazole having one of A¹ and A² at position 1 and the other at position 3 (hereinafter Y-15); and
(2) wherein A¹ and A² are defined as follows:
   A¹ or A² is a phenyl that may be optionally substituted by one or more substituents selected from the group consisting of a hydroxy, a lower alkoxy, a lower alkyl, a lower thioalkyl, an amino optionally substituted by a lower alkyl, a halogen, a phenyl and a thiadiazolyl (hereinafter A¹ or A² is regarded as A-1);
   A¹ or A² is a furyl, thiazolyl, thienyl or pyrazolyl, each of which may be optionally substituted by one of more substituents selected from the group consisting of a lower alkyl optionally substituted by a halogen, a lower alkylsulfonyl, a lower alkylcarbamoyl, a nitro, a phenyl, a benzoyl, and a thienyl (hereinafter A¹ or A² is regarded as A-2);
   A¹ or A² is a pyridyl that may be optionally substituted by a halogen (hereinafter A¹ or A² is regarded as A-3);
   A¹ or A² is a benzofuryl or a indolyl (hereinafter A¹ or A² is regarded as A-4);
   Both A¹ and A² are A-1 (hereinafter A¹ and A² are regarded as A-5);
   One of A¹ and A² is A-1 and the other is A-2 (hereinafter A¹ and A² are regarded as A-6);
   One of A¹ and A² is A-1 and the other is A-3 (hereinafter A¹ and A² are regarded as A-7);
   One of A¹ and A² is A-1 and the other is A-4 (hereinafter A¹ and A² are regarded as A-8);
   Both of A¹ and A² are A-2 (hereinafter A¹ and A² are regarded as A-9);
   One of A¹ and A² is A-2 and the other is A-3 (hereinafter A¹ and A² are regarded as A-10); and
(3) wherein Z is defined as follows:
   Z is a single bond; or
   Z is -N- or -HC=CH-.

More preferable compounds having one group: A¹ and one group: A² are those of formula (I) wherein Z is a single bond; and a combination of the 5-membered ring comprising Y¹, Y², Y³, Y⁴ and Y⁵, and A¹ and A², i.e., (Y, A) is as follows:
(Y-1, A-5), (Y-2, A-5), (Y-3, A-5), (Y-4, A-5), (Y-5, A-5), (Y-6, A-5), (Y-7, A-5), (Y-8, A-5), (Y-9, A-5), (Y-10, A-5), (Y-11, A-5), (Y-12, A-5), (Y-13, A-5), (Y-14, A-5), (Y-1, A-6), (Y-2, A-6), (Y-3, A-6), (Y-4, A-6), (Y-5, A-6), (Y-6, A-6), (Y-7, A-6), (Y-8, A-6), (Y-9, A-6), (Y-10, A-6), (Y-11, A-6), (Y-12, A-6), (Y-13, A-6), (Y-14, A-6), (Y-1, A-7), (Y-2, A-7), (Y-3, A-7), (Y-4, A-7), (Y-5, A-7), (Y-6, A-7), (Y-7, A-7), (Y-8, A-7), (Y-9, A-7), (Y-10, A-7), (Y-11, A-7), (Y-12, A-7), (Y-13, A-7), (Y-14, A-7), (Y-1, A-8), (Y-2, A-8), (Y-3, A-8), (Y-4, A-8), (Y-5, A-8), (Y-6, A-8), (Y-7, A-8), (Y-8, A-8), (Y-9, A-8), (Y-10, A-8), (Y-11, A-8), (Y-12, A-8), (Y-13, A-8), (Y-14, A-8), (Y-1, A-9), (Y-2, A-9), (Y-3, A-9), (Y-4, A-9), (Y-5, A-9), (Y-6, A-9), (Y-7, A-9), (Y-8, A-9), (Y-9, A-9), (Y-10, A-9), (Y-11, A-9), (Y-12, A-9), (Y-13, A-9), (Y-14, A-9), (Y-1, A-10), (Y-2, A-10), (Y-3, A-10), (Y-4, A-10), (Y-5, A-10), (Y-6, A-10), (Y-7, A-10), (Y-8, A-10), (Y-9, A-10), (Y-10, A-10), (Y-11, A-10), (Y-12, A-10), (Y-13, A-10) or (Y-14, A-10).

Some illustrative examples of compound (I) according to the invention are shown in Tables below.

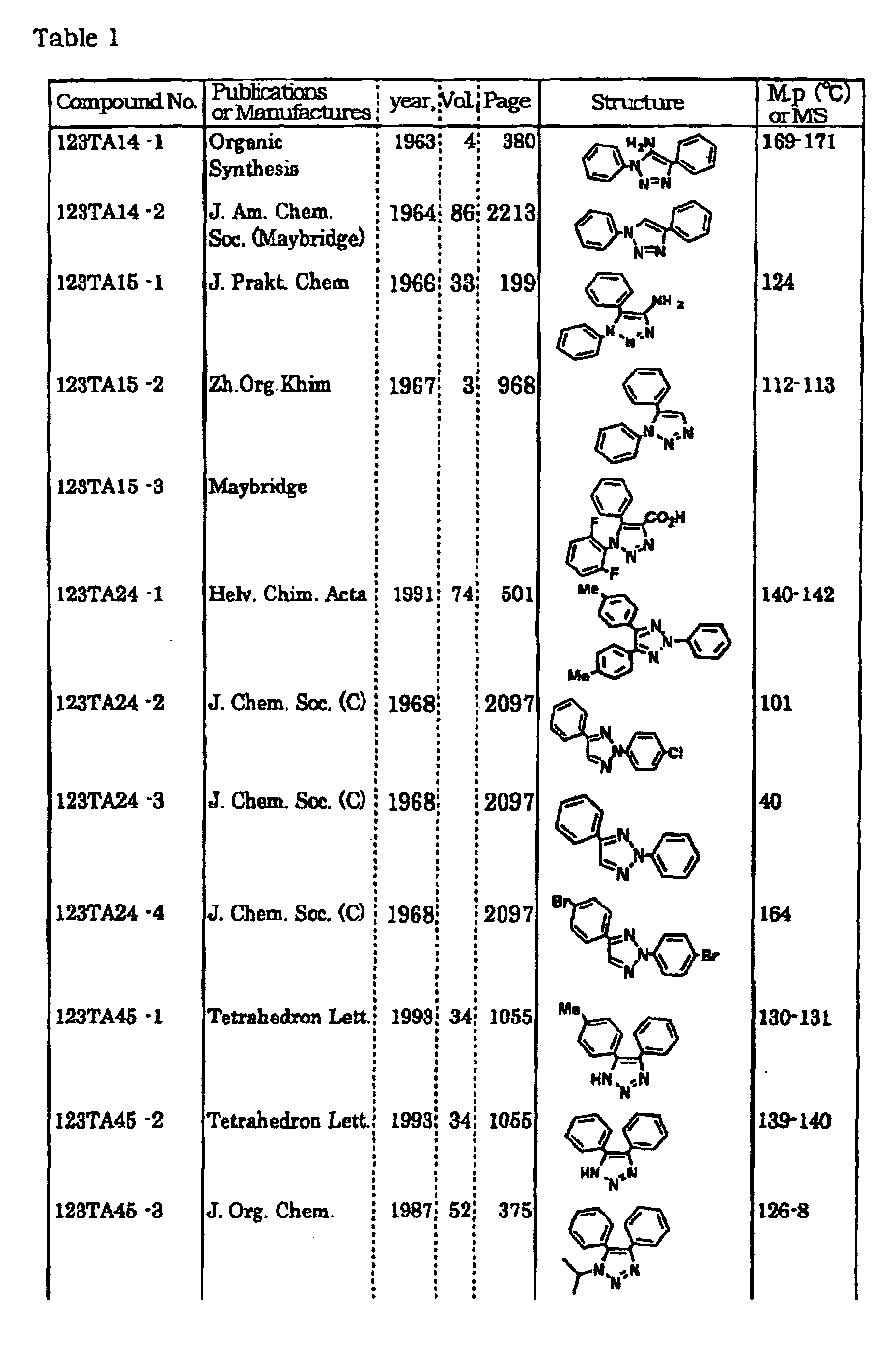

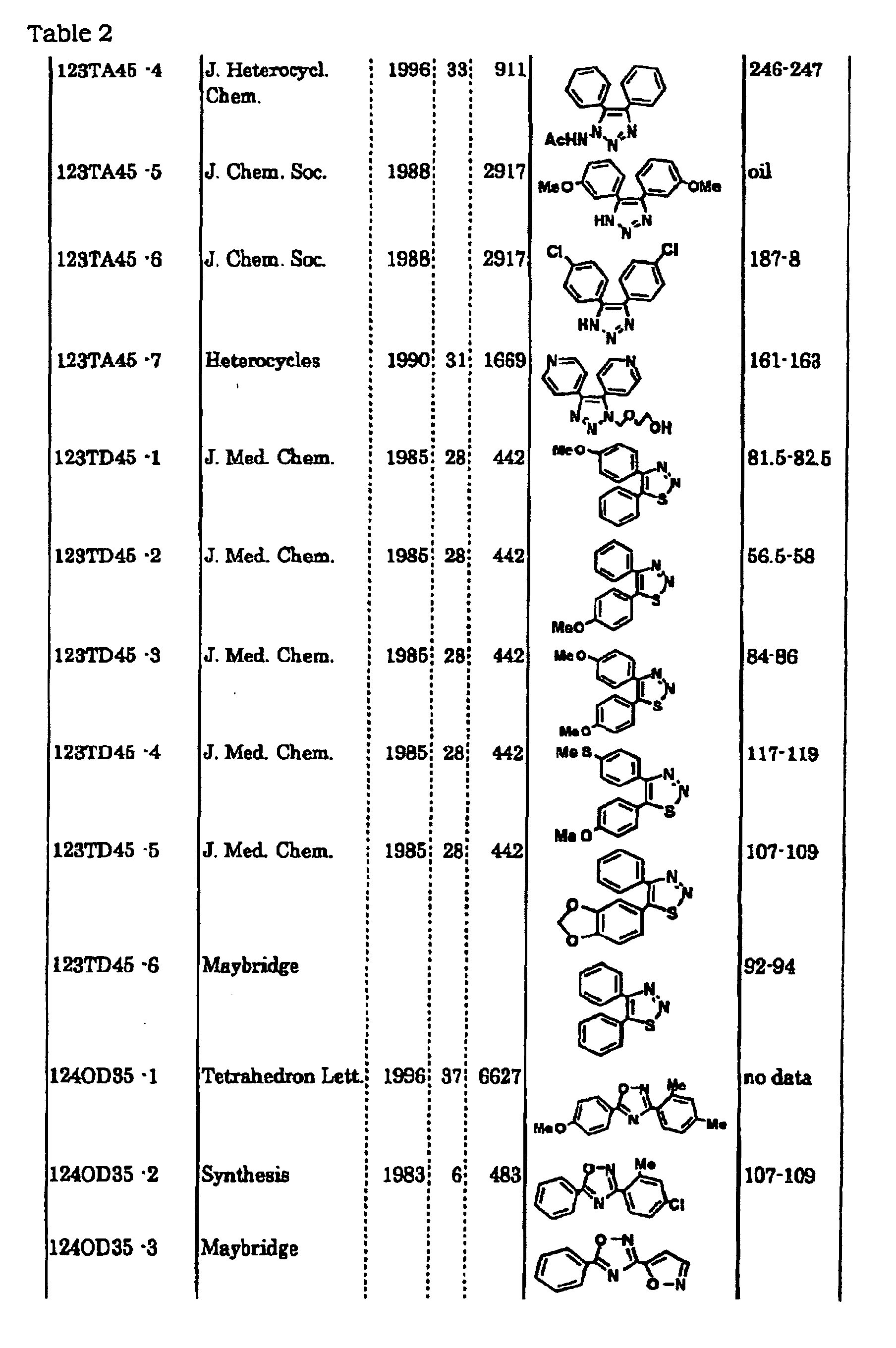

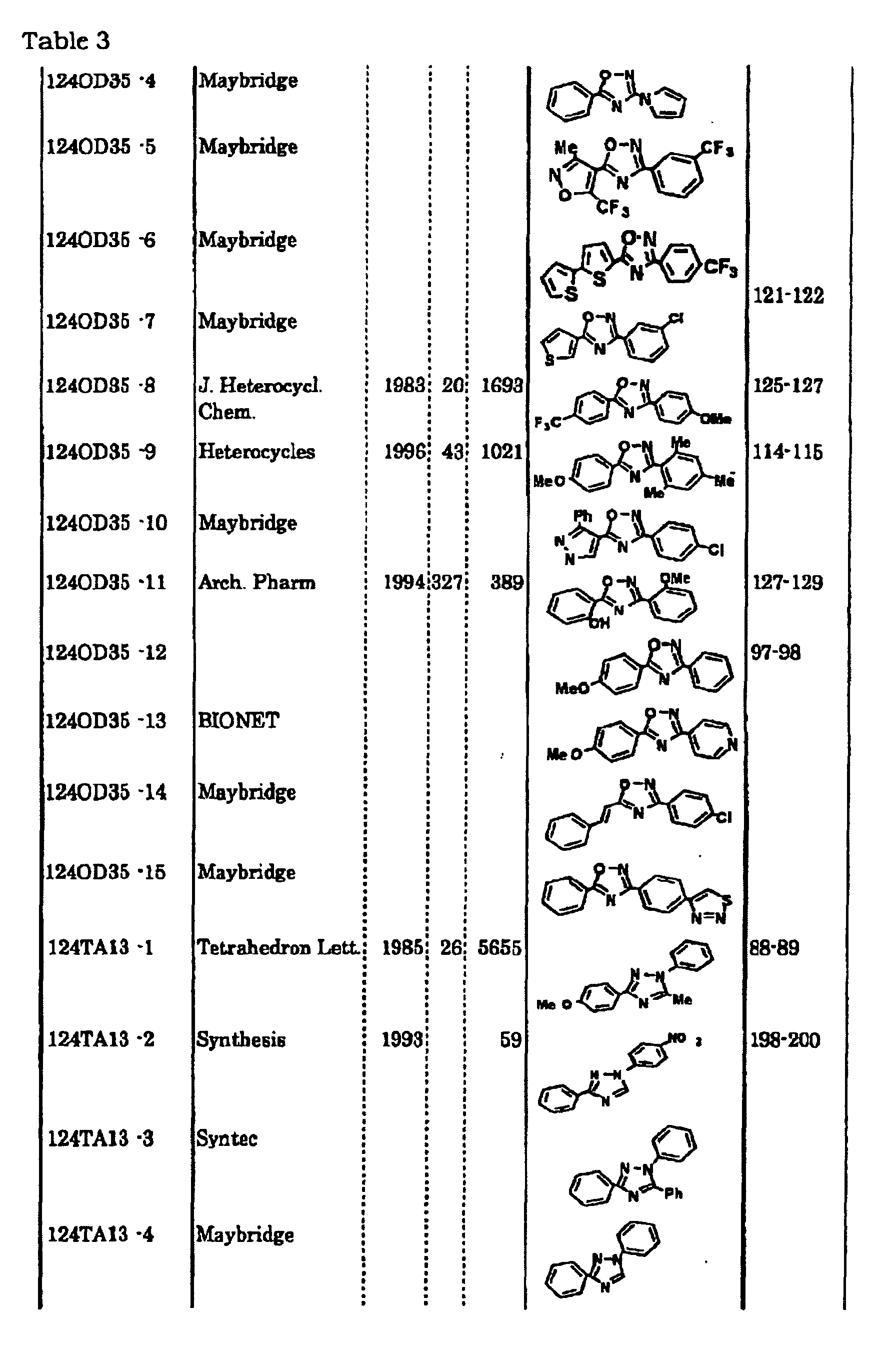

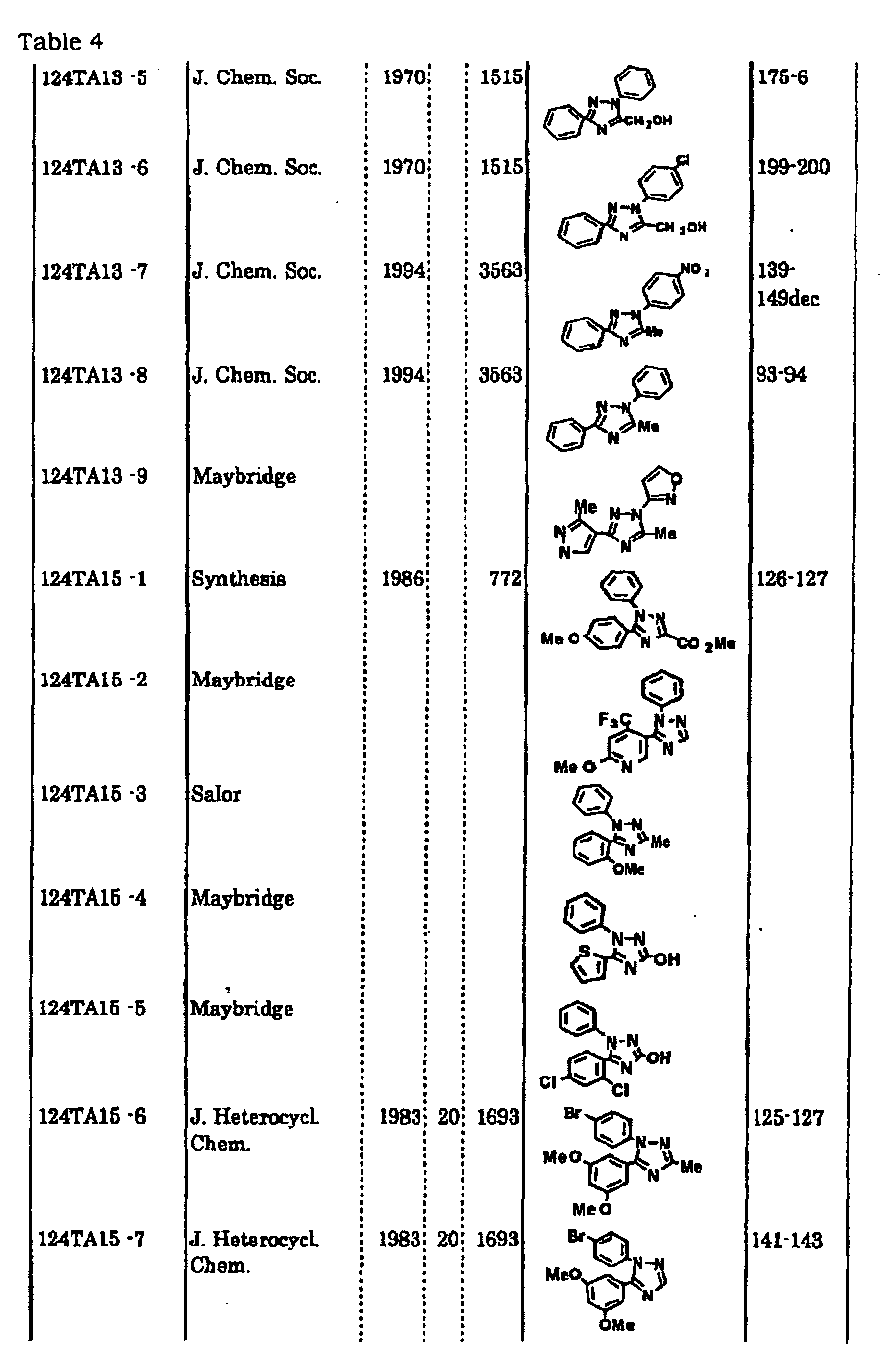

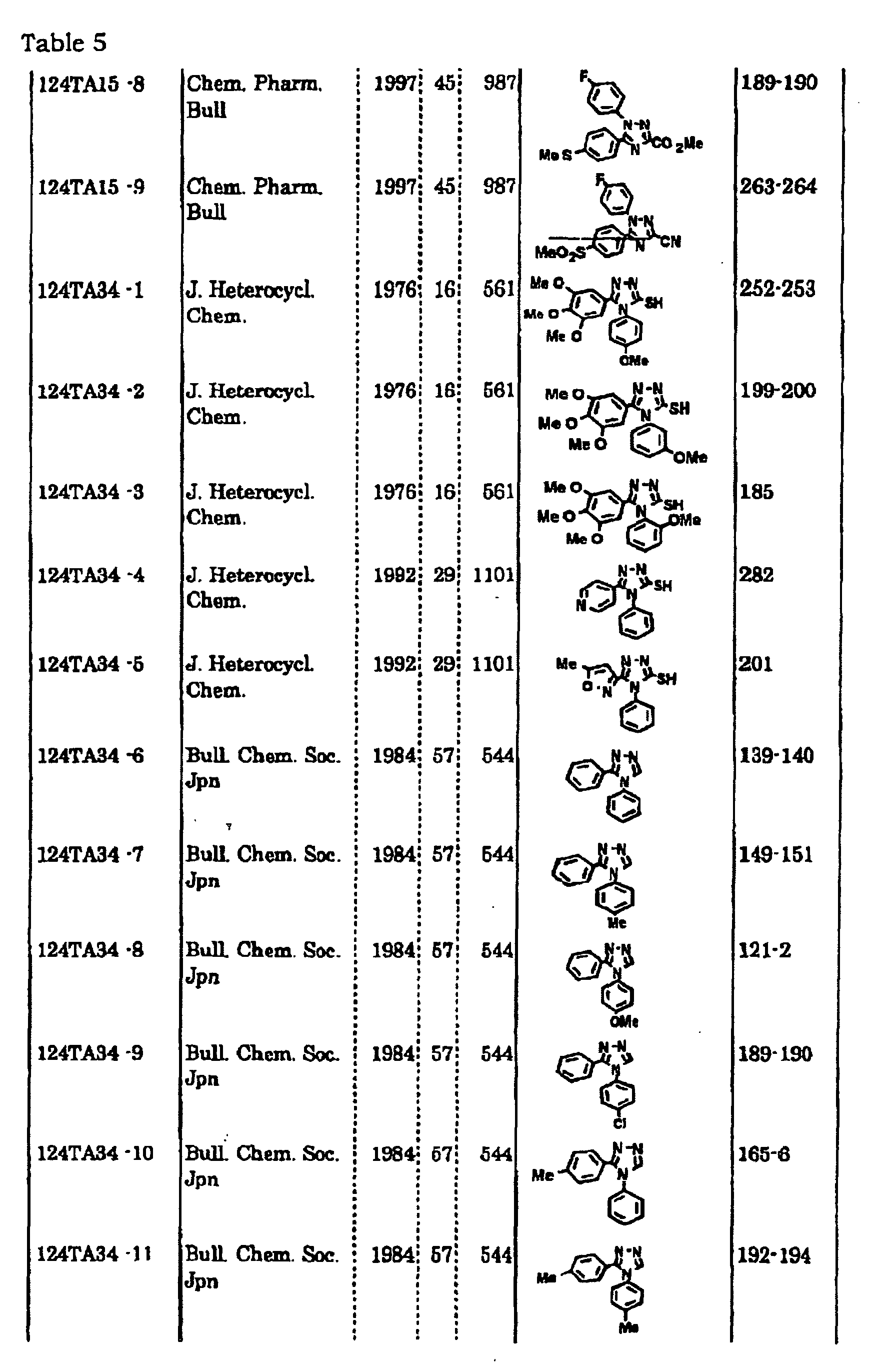

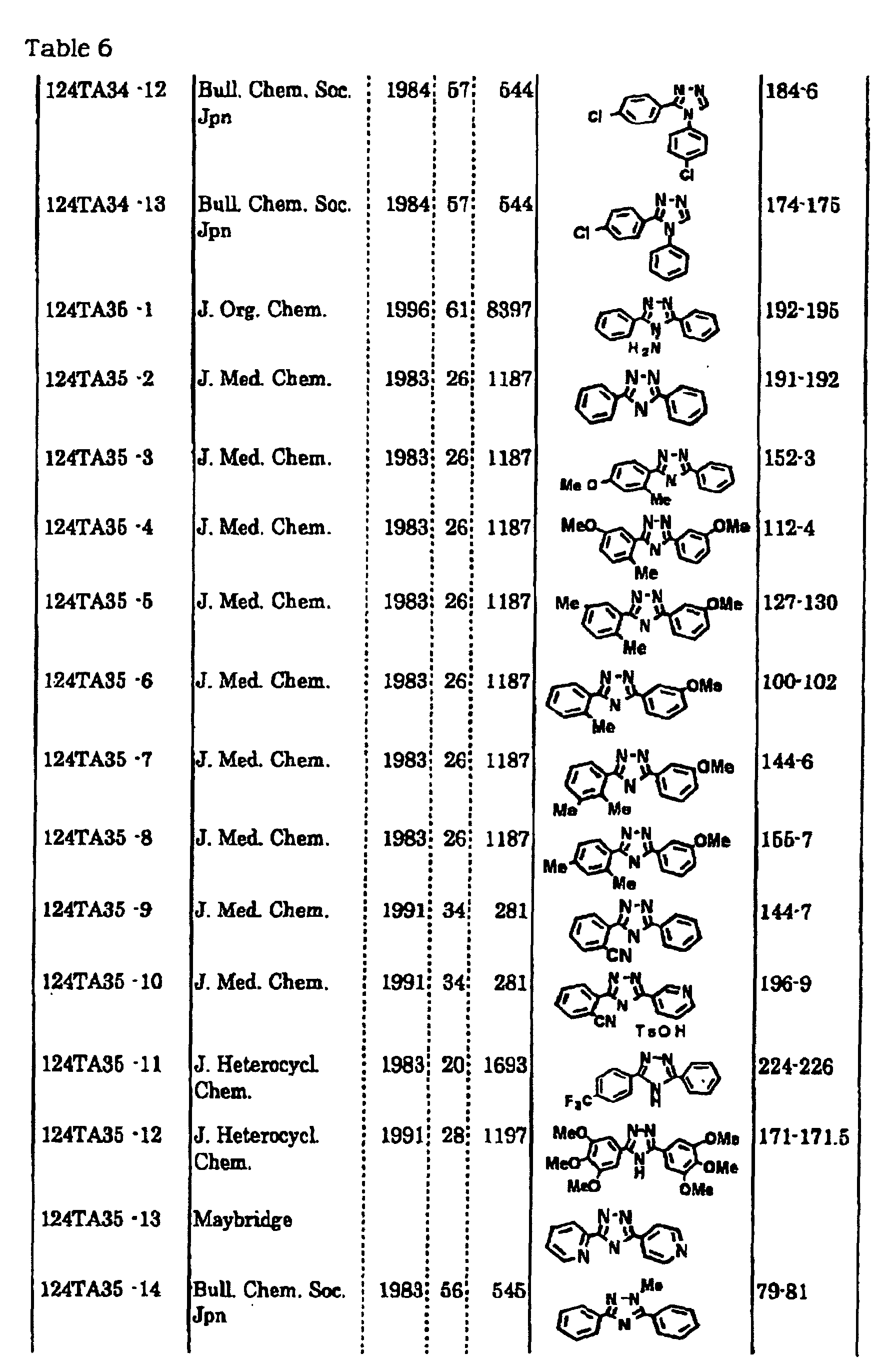

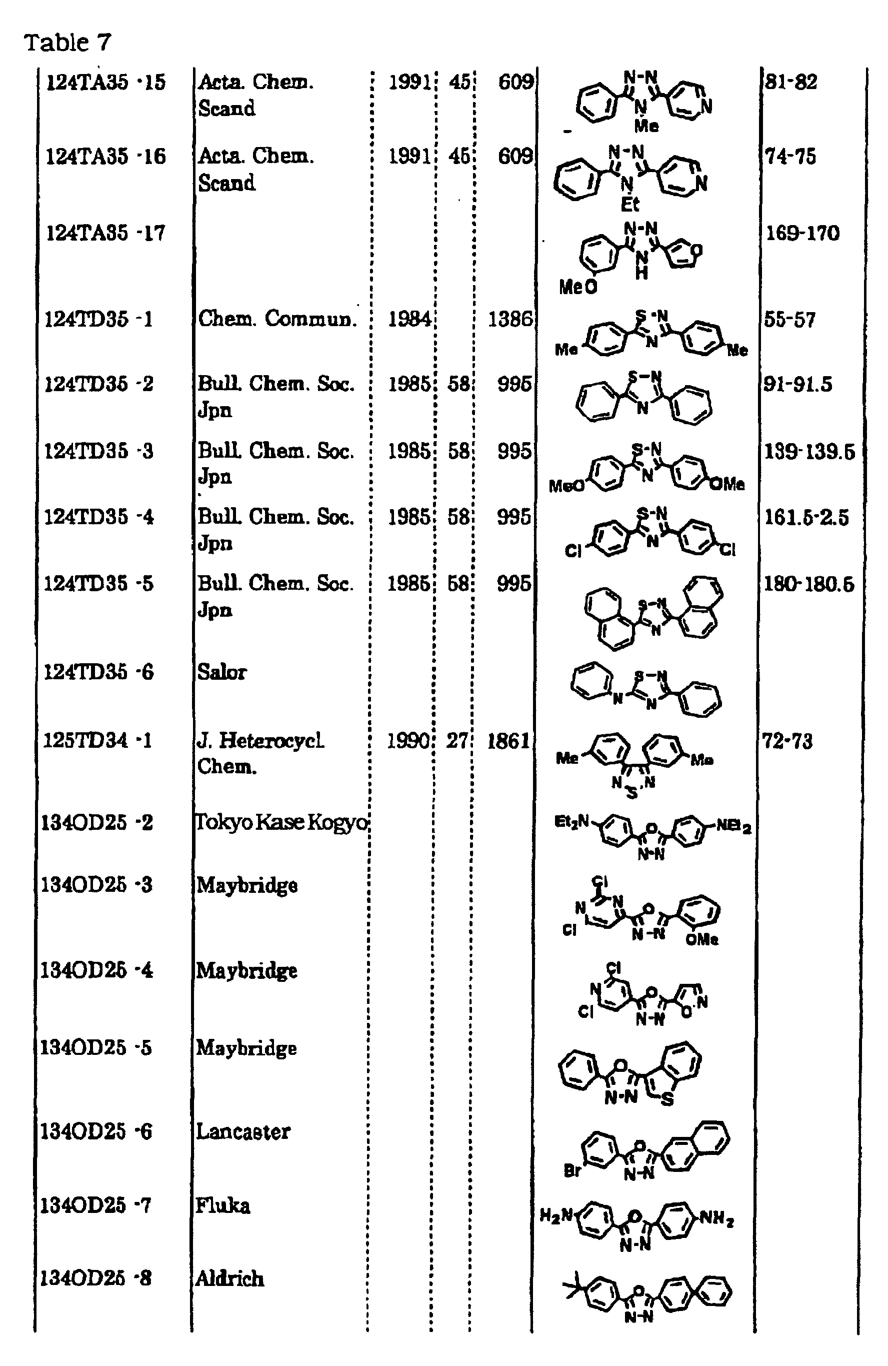

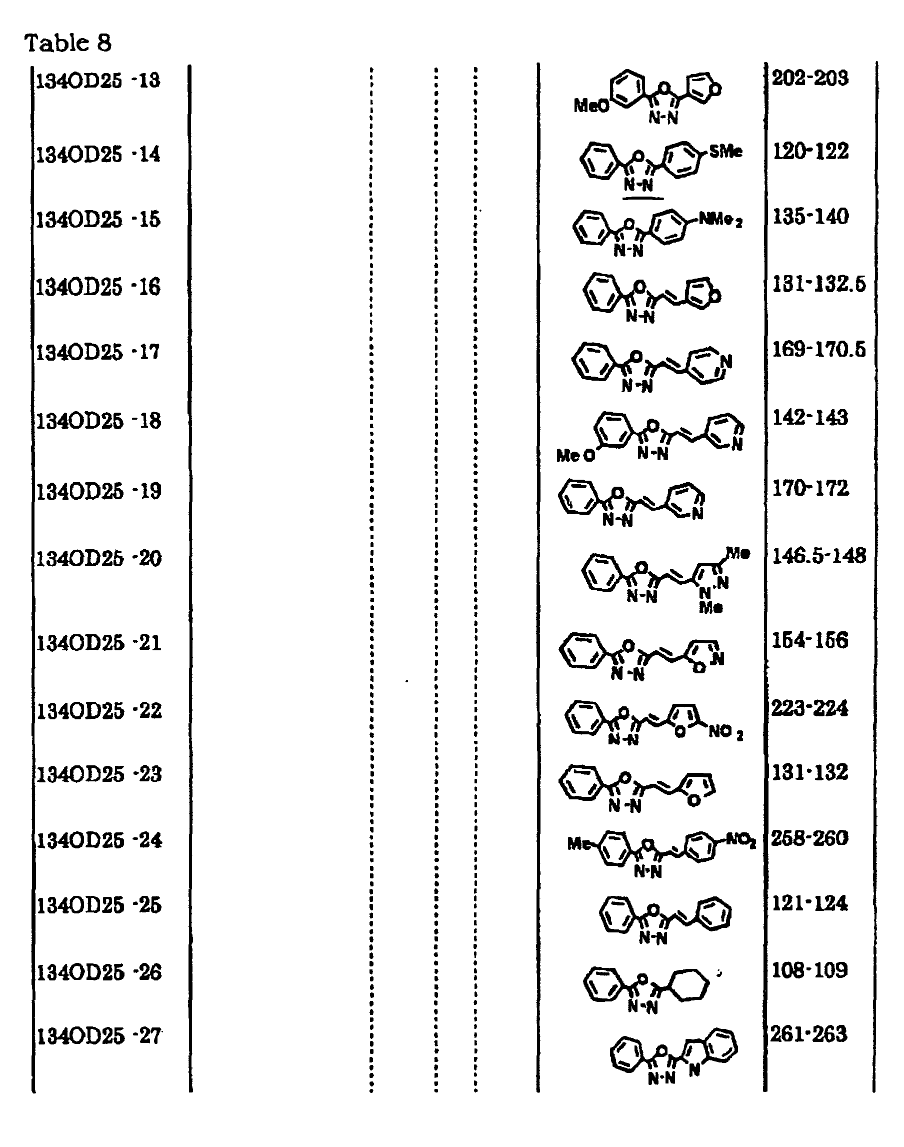

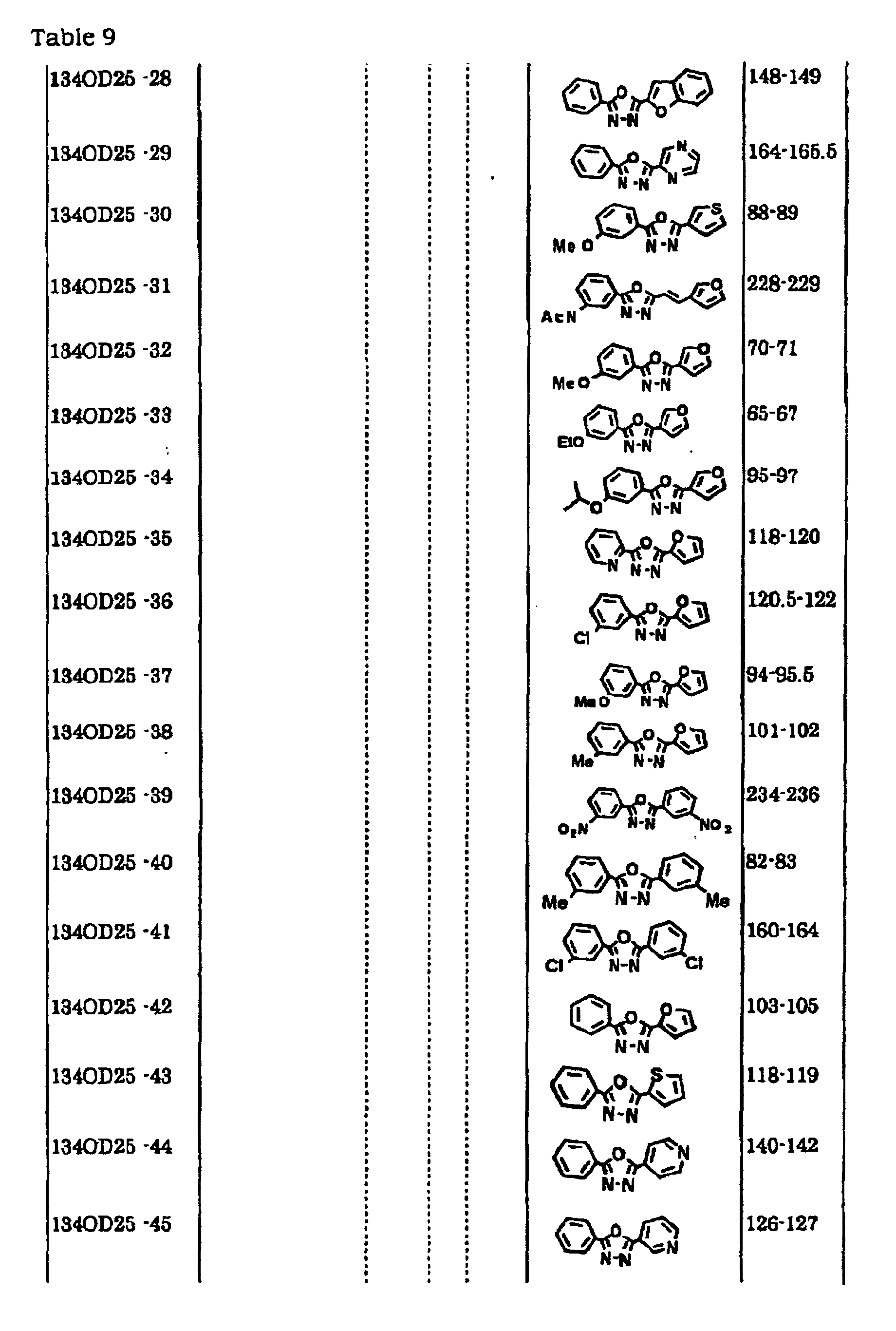

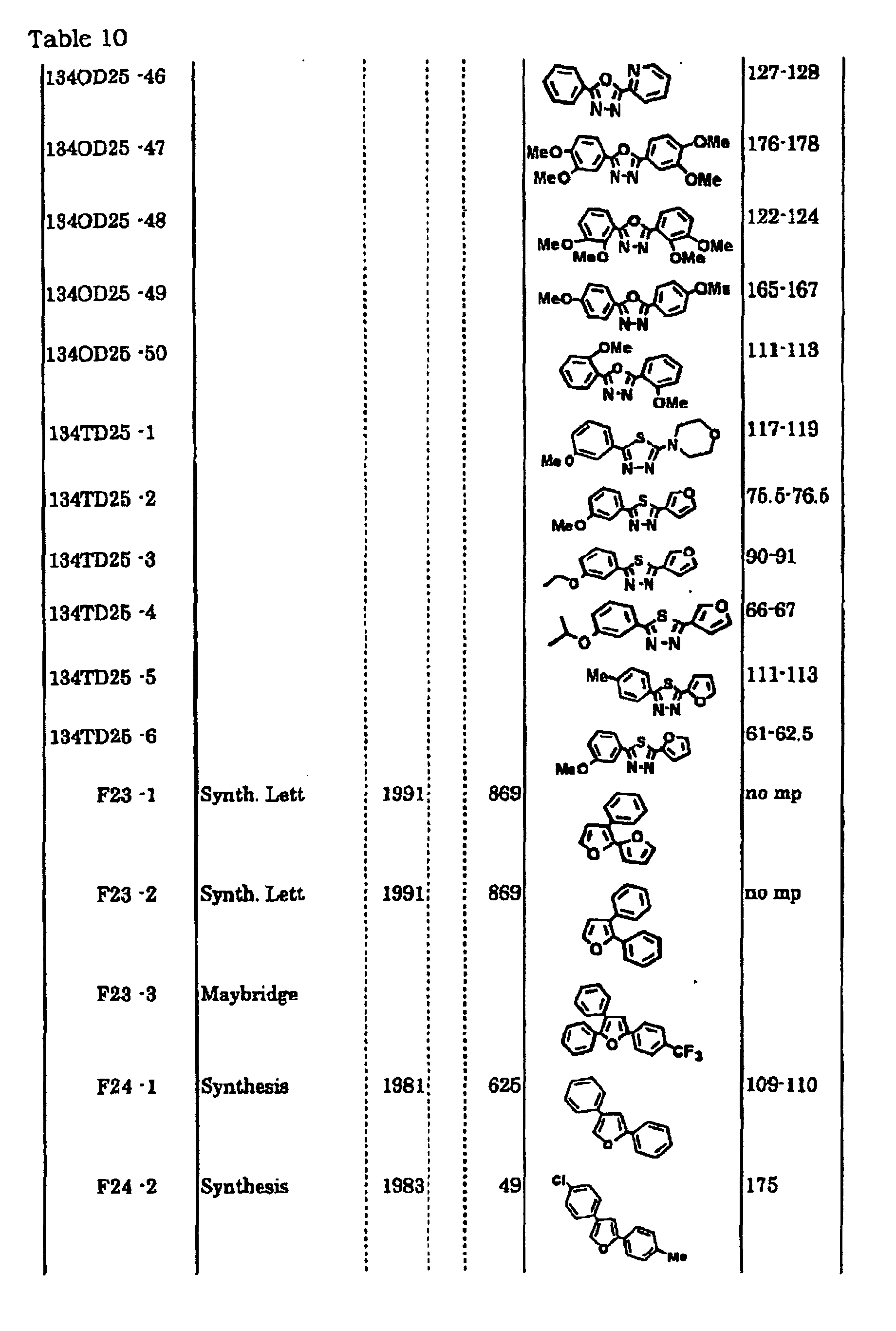

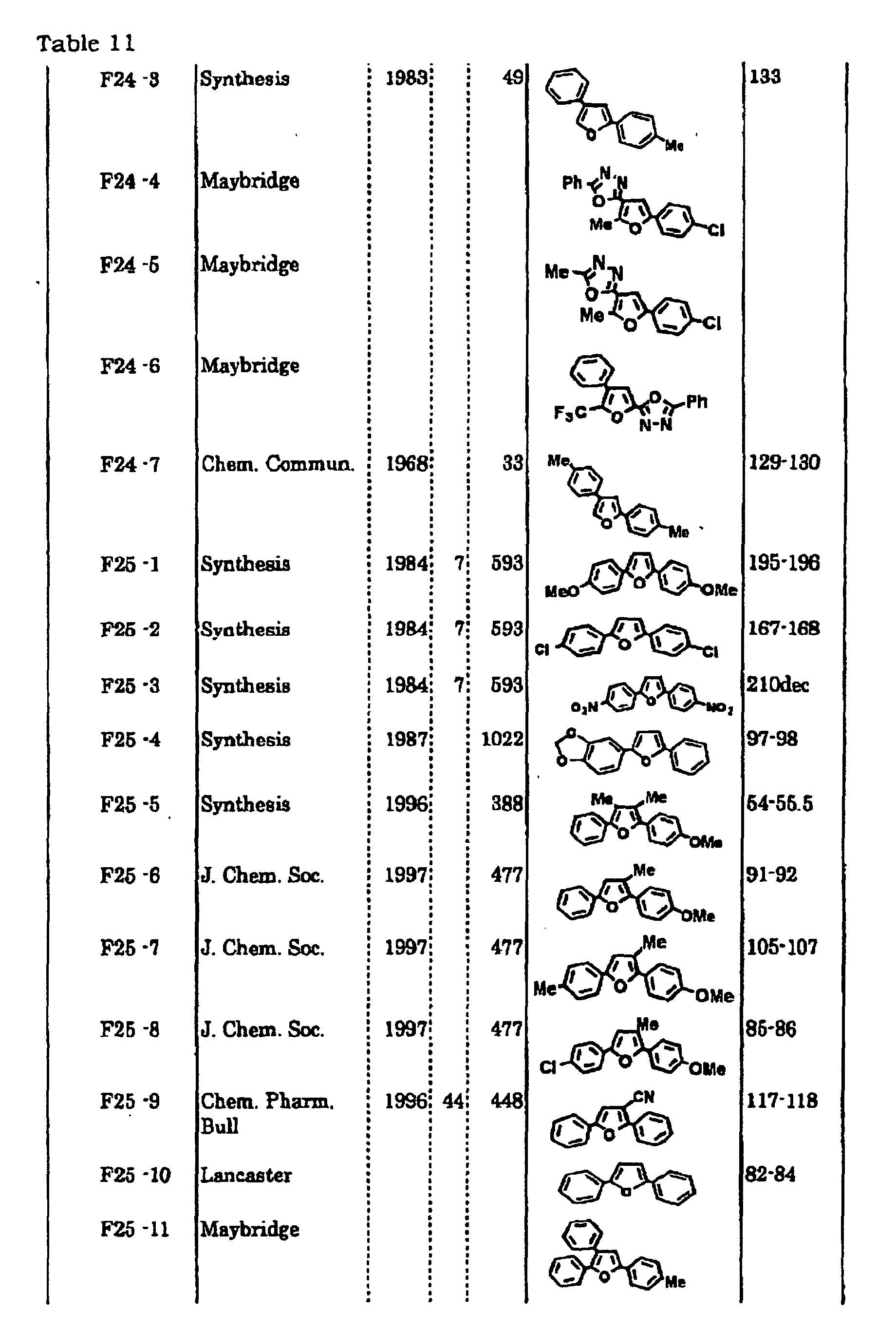

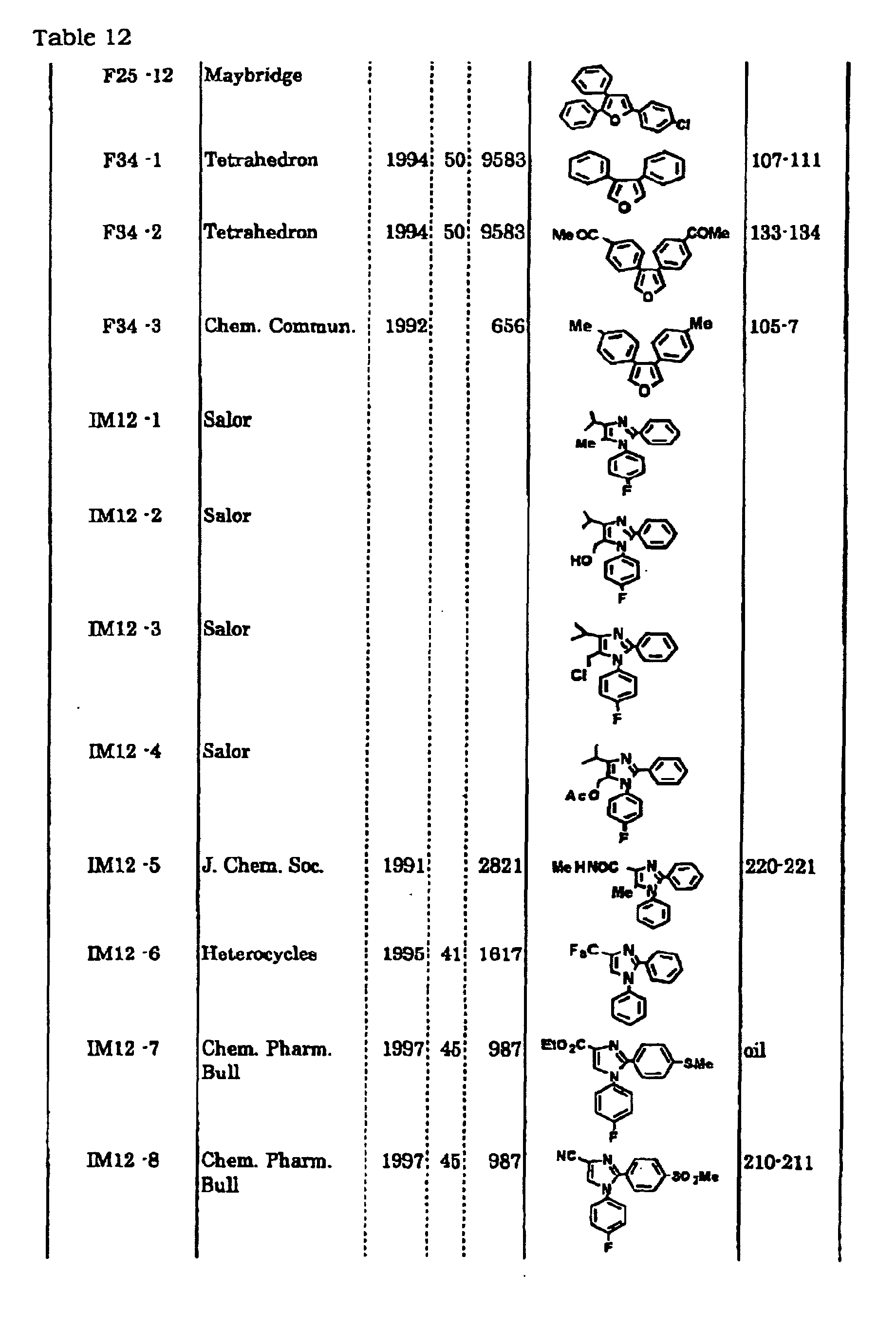

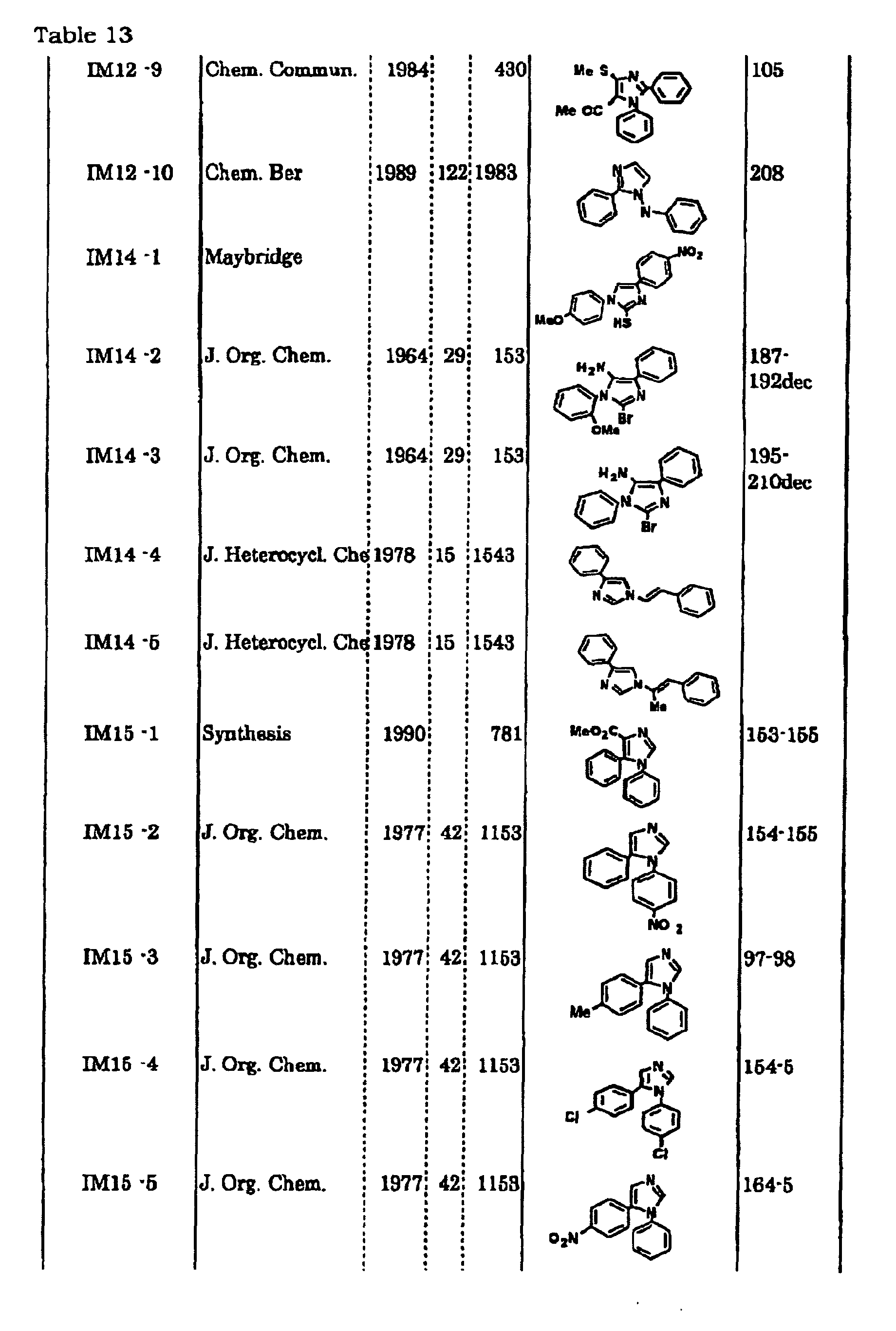

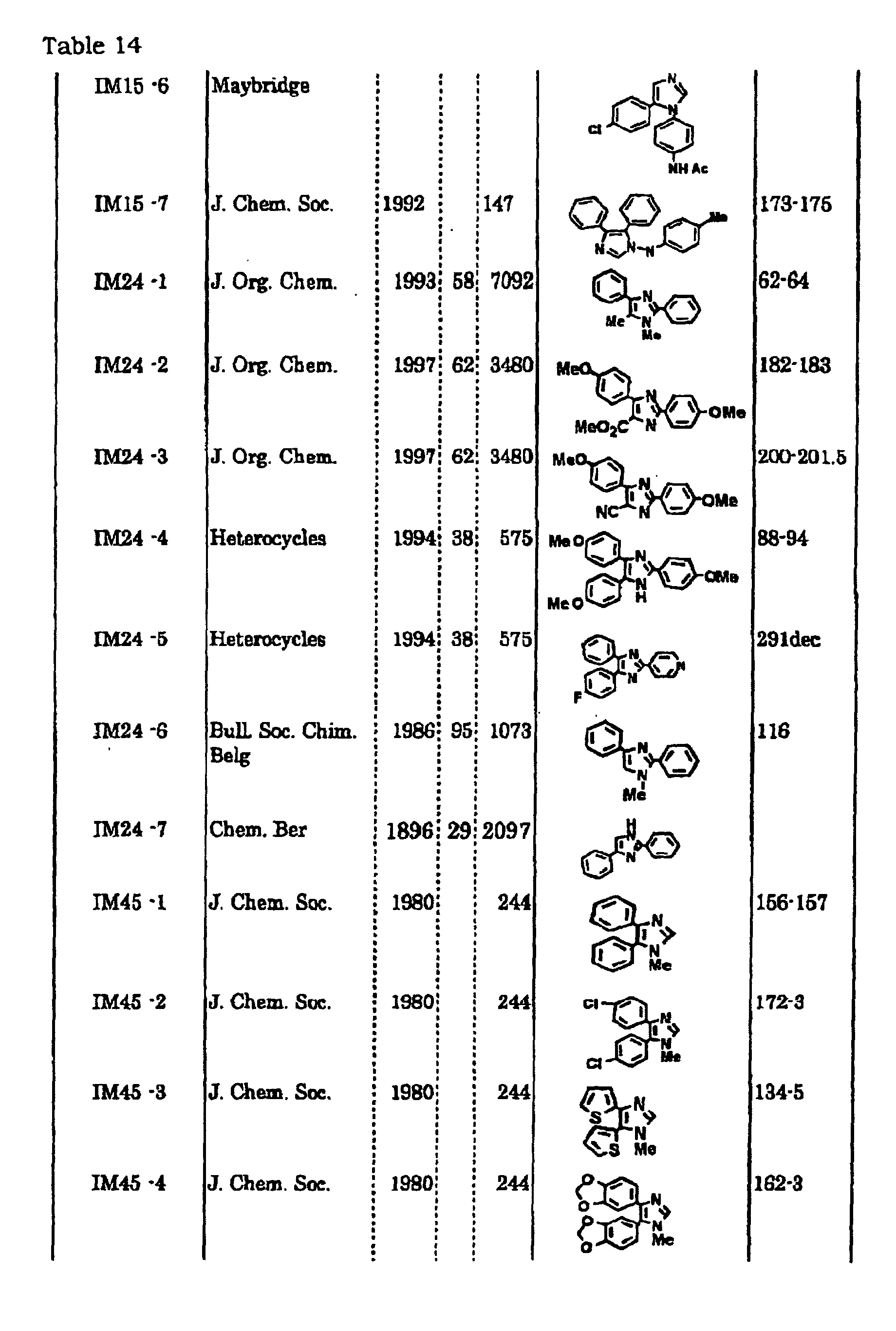

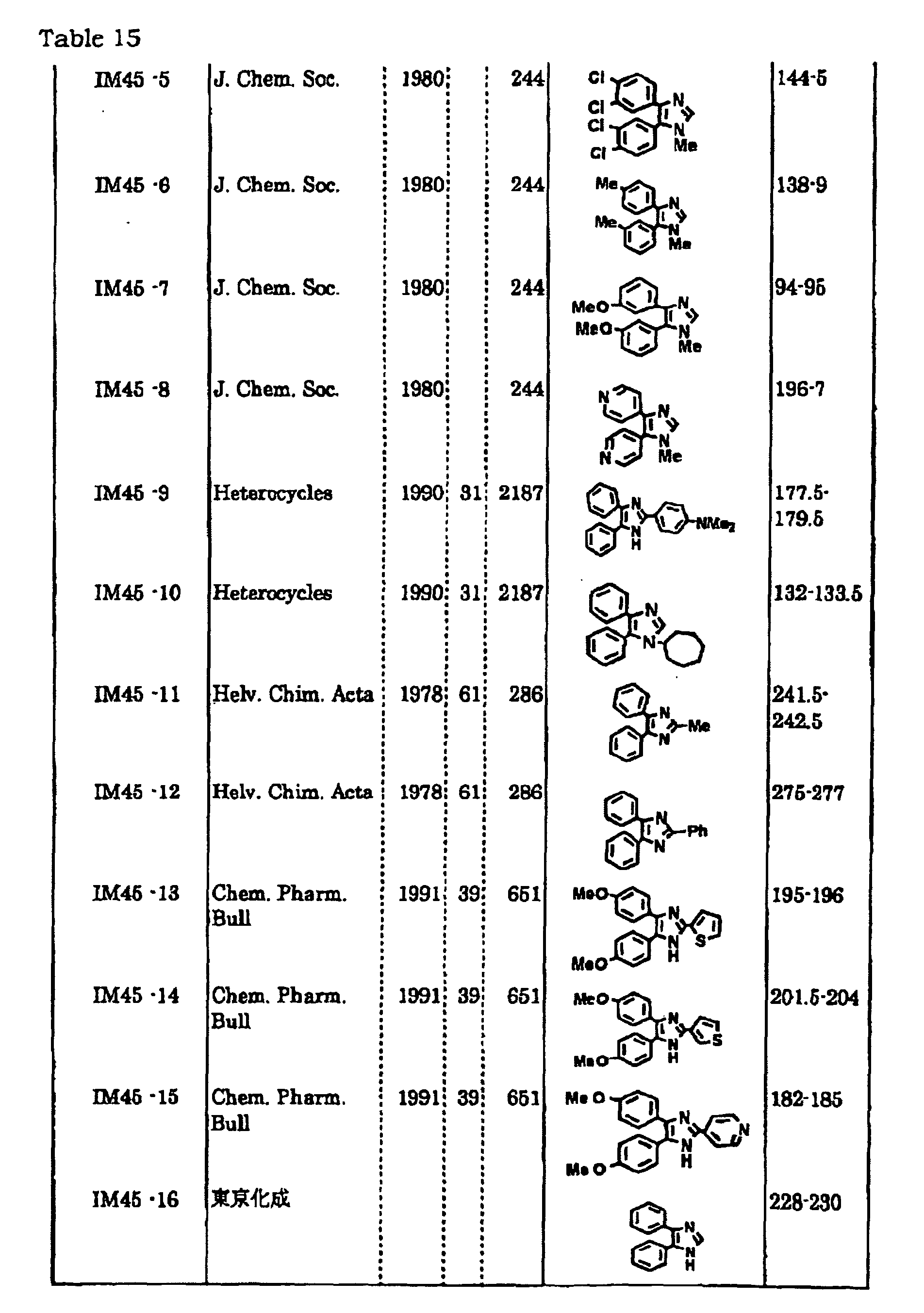

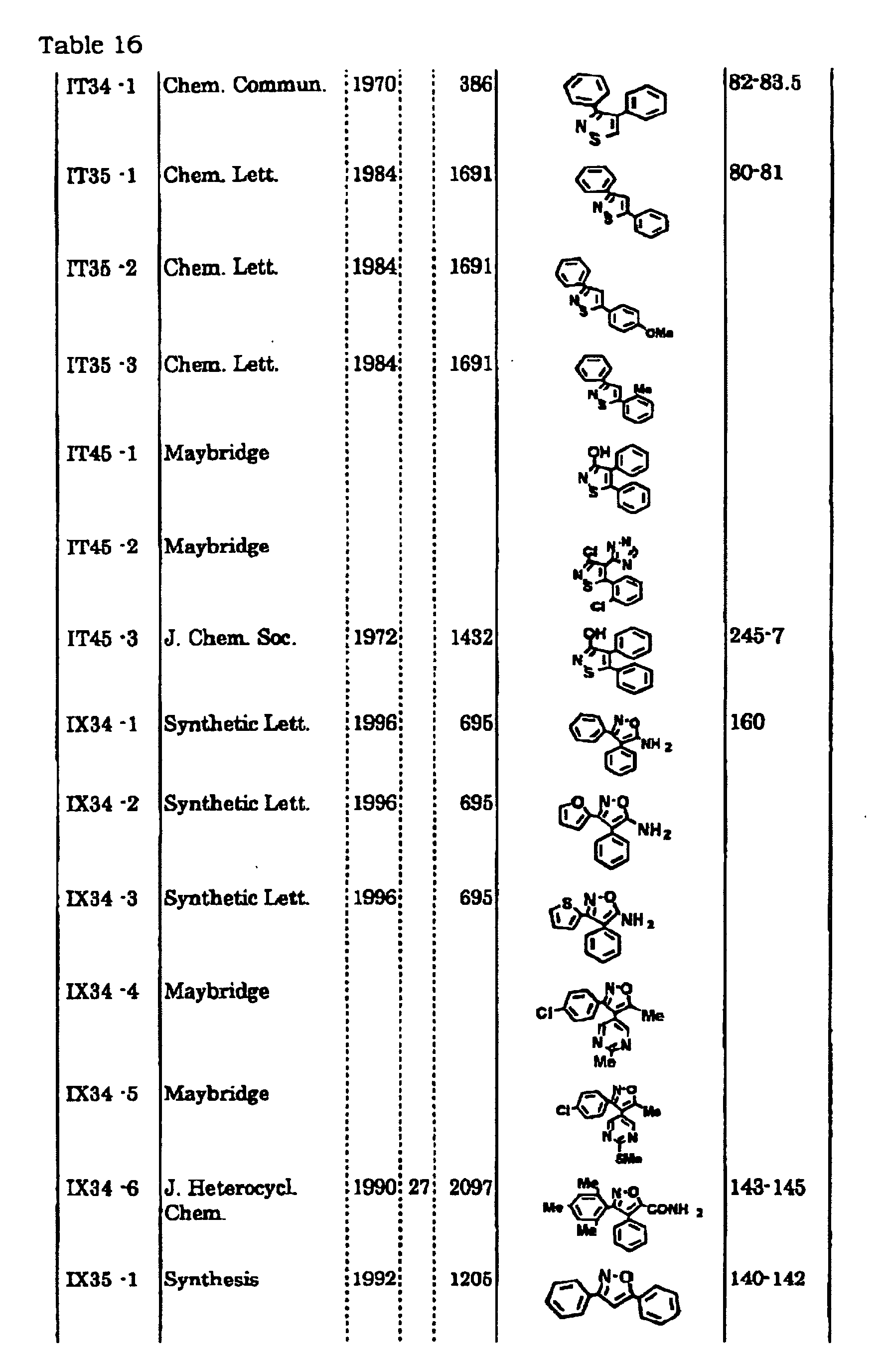

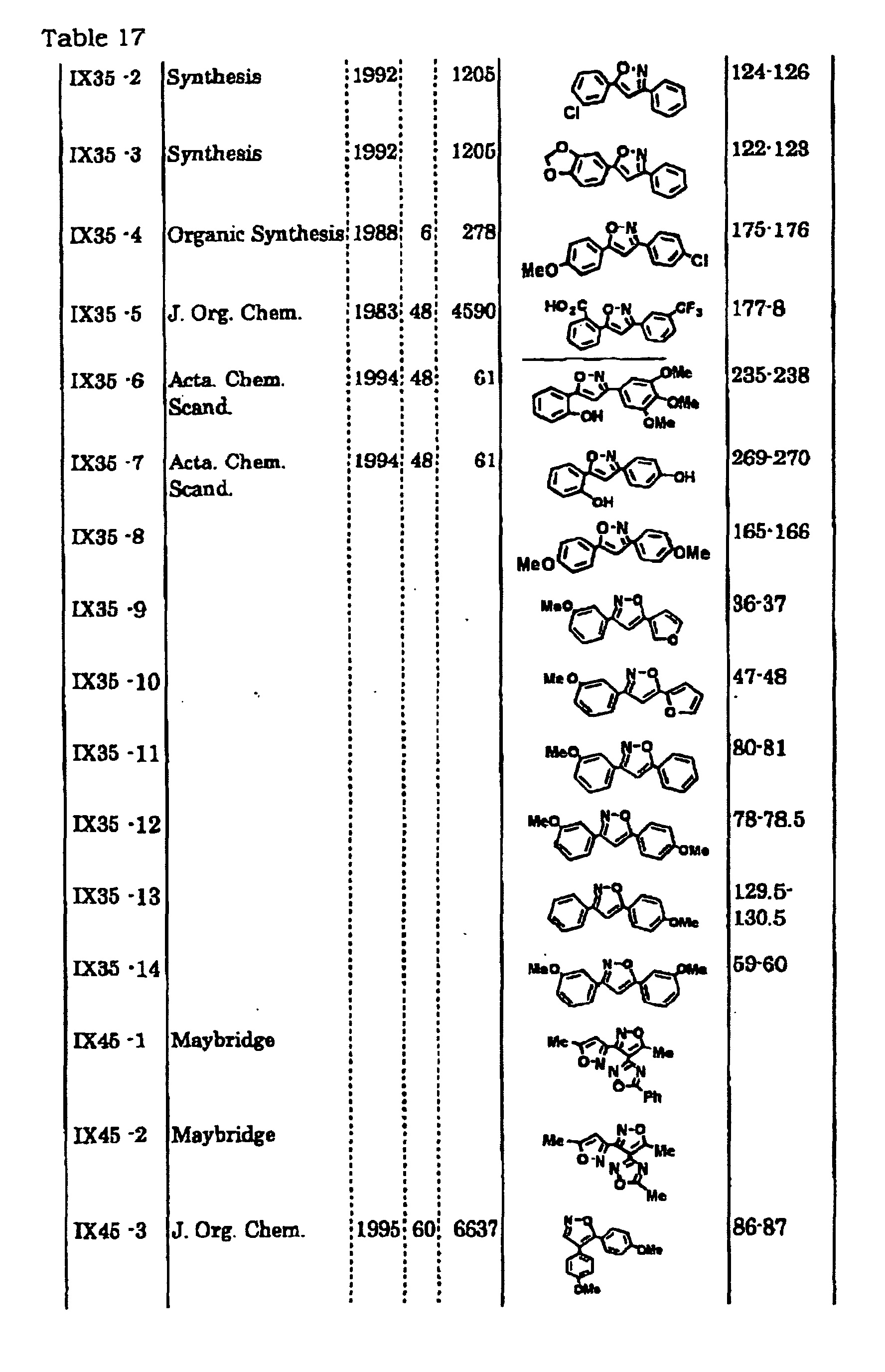

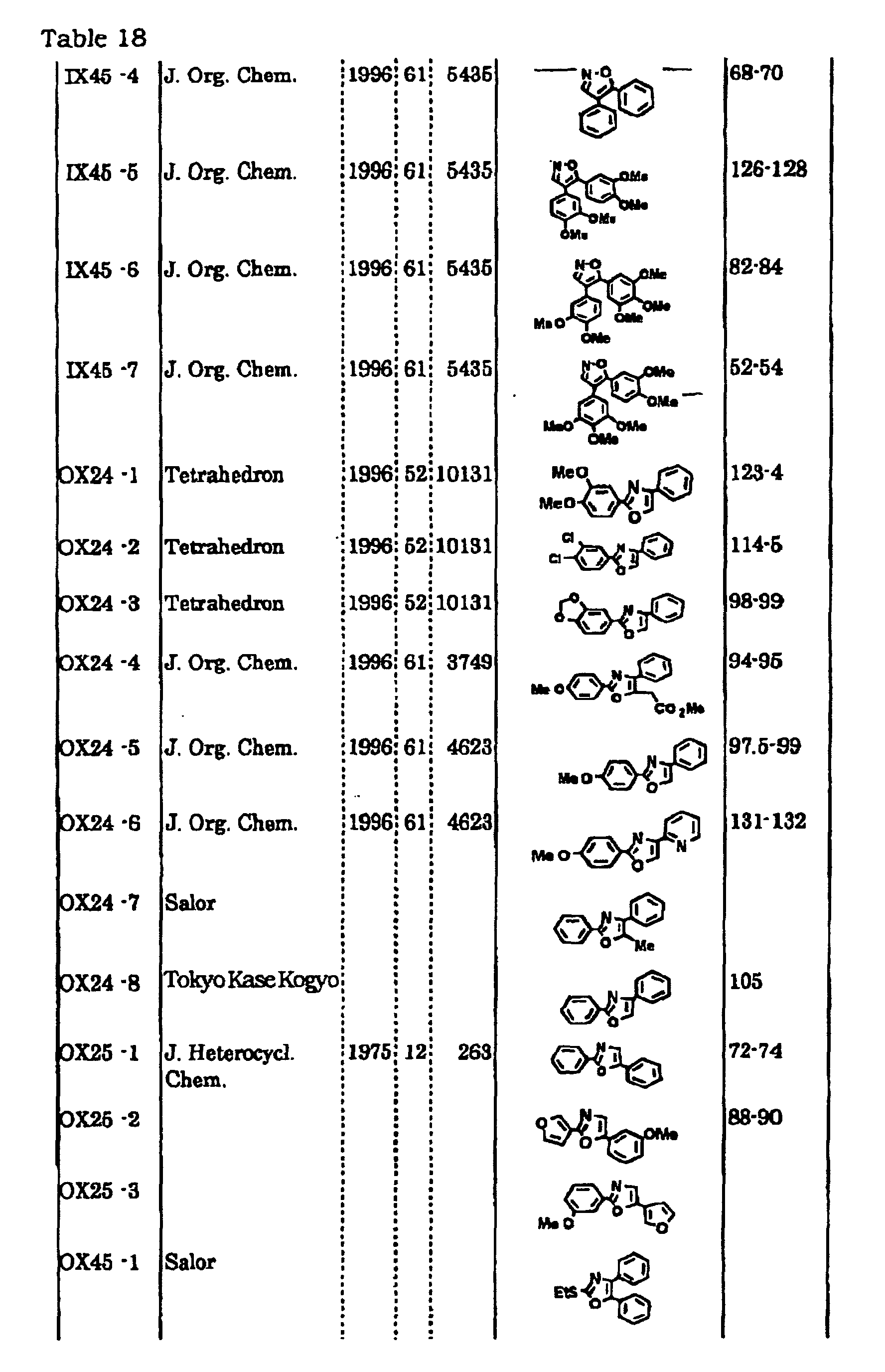

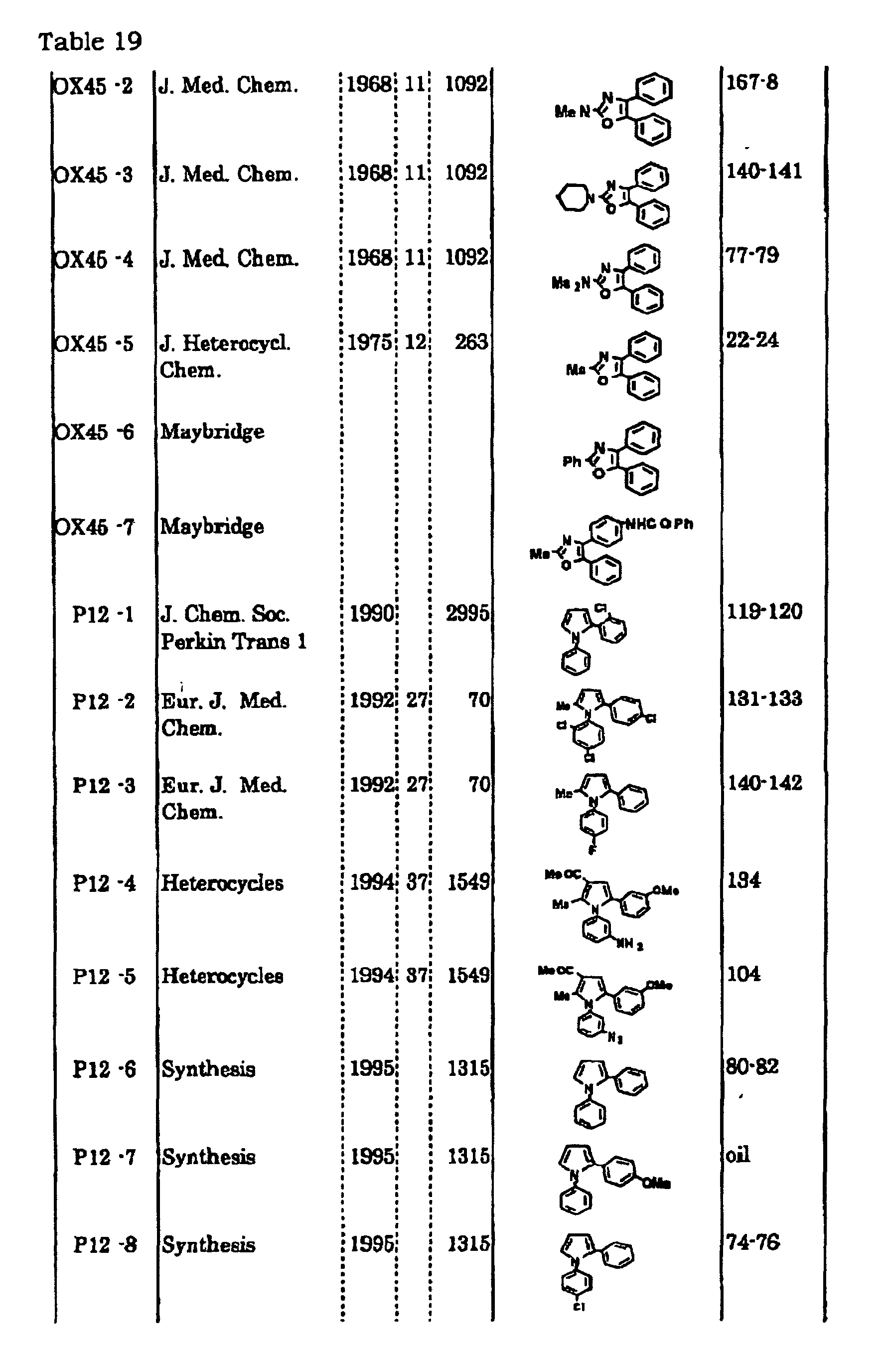

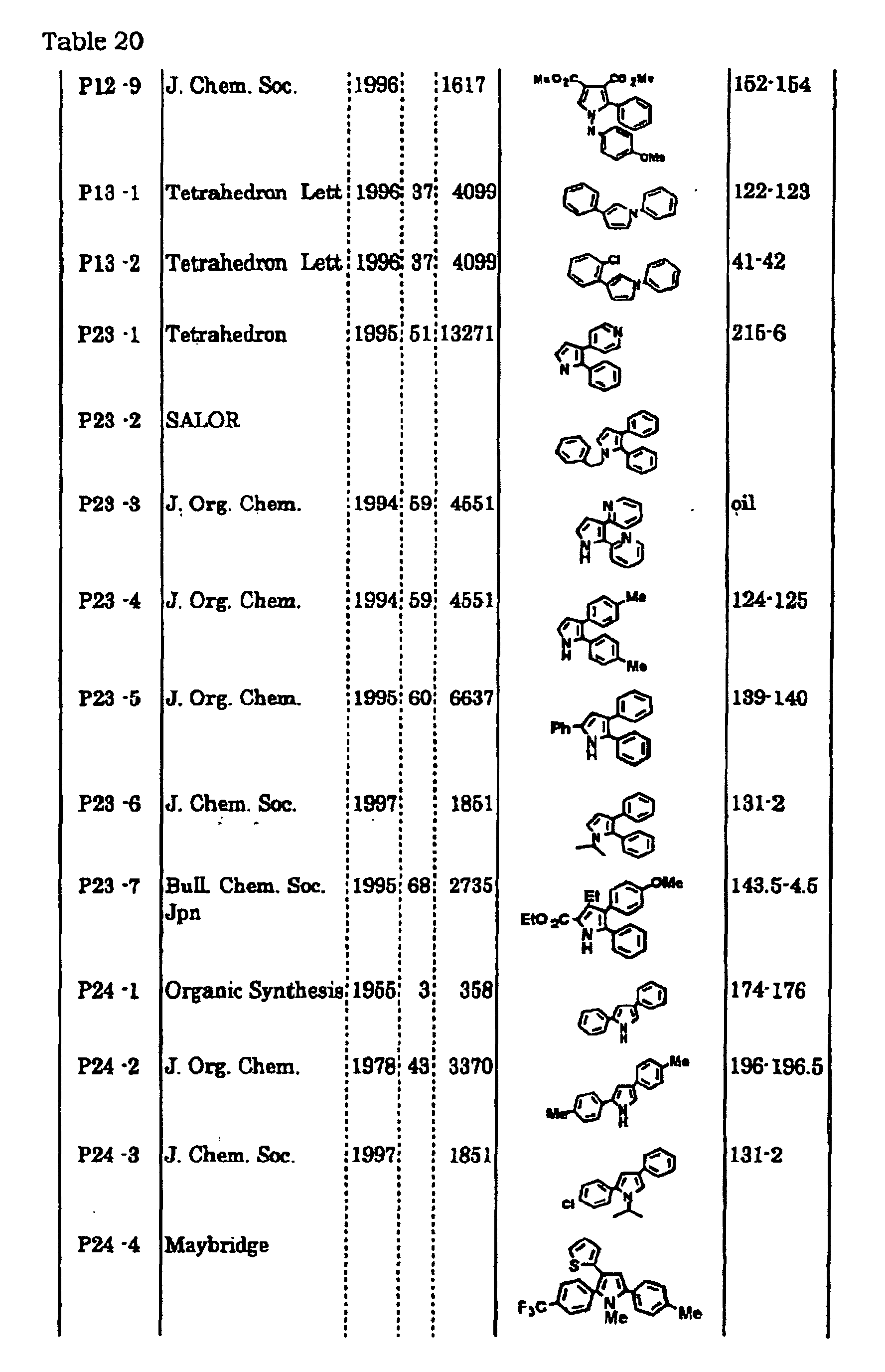

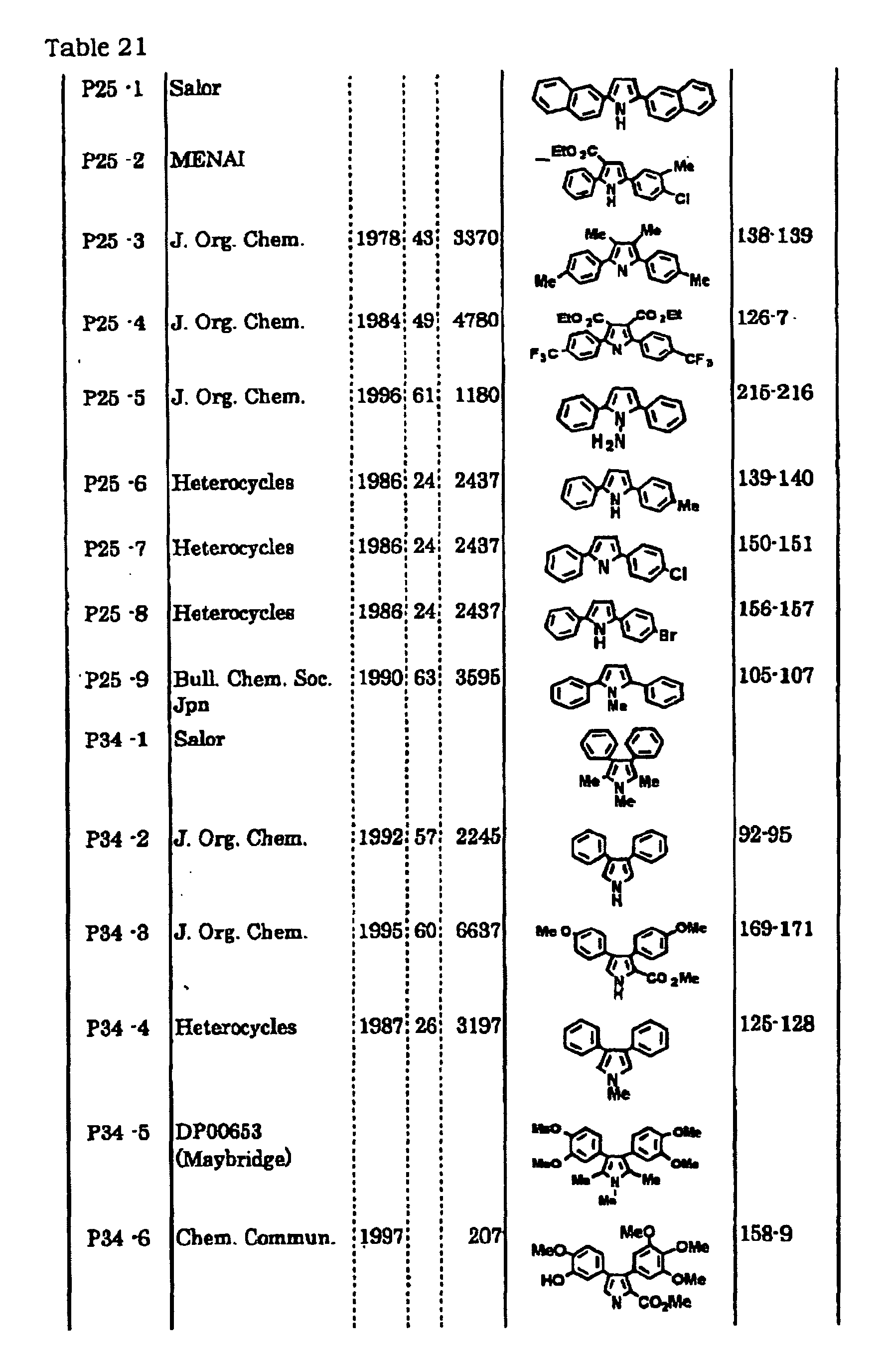

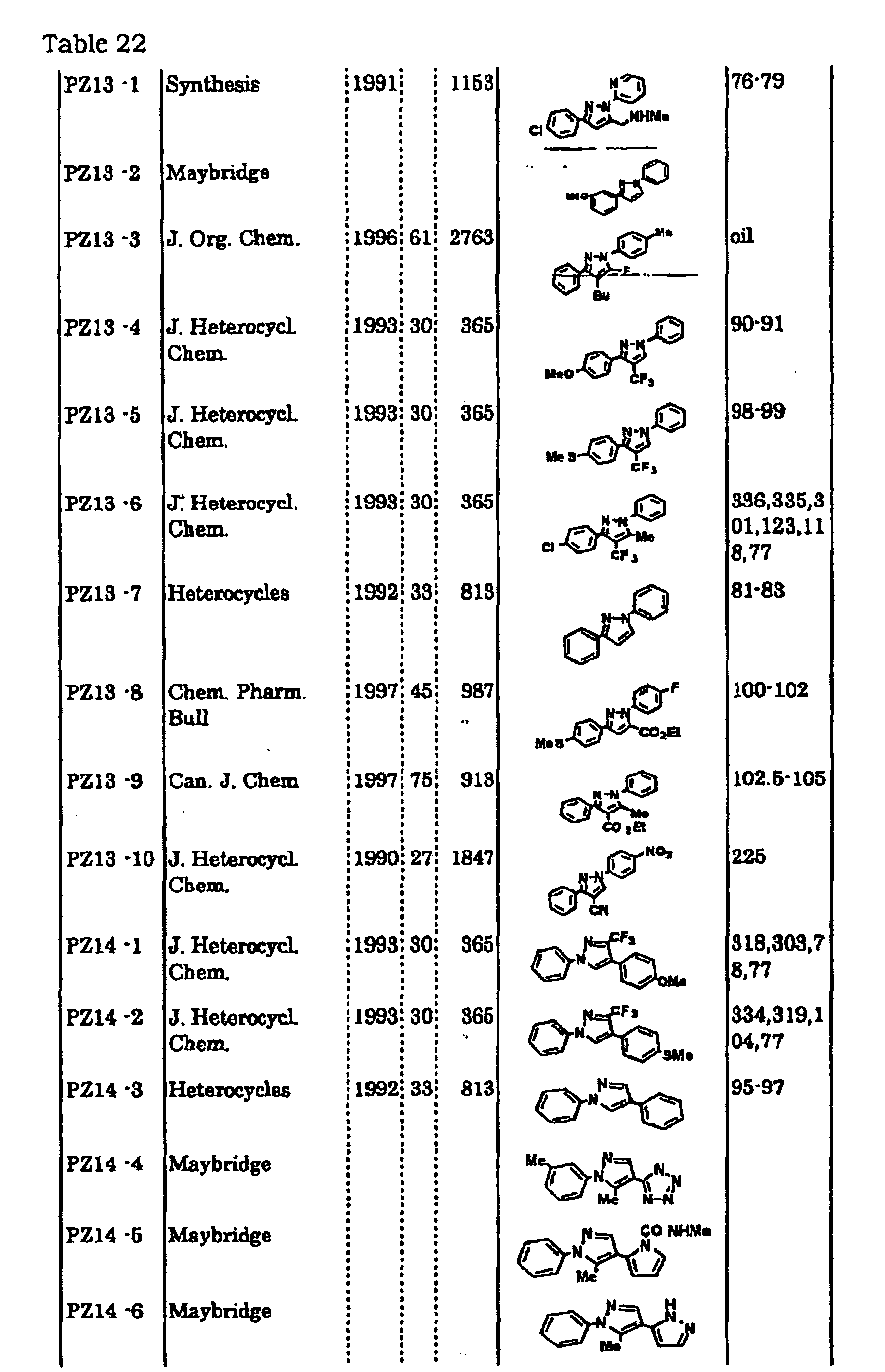

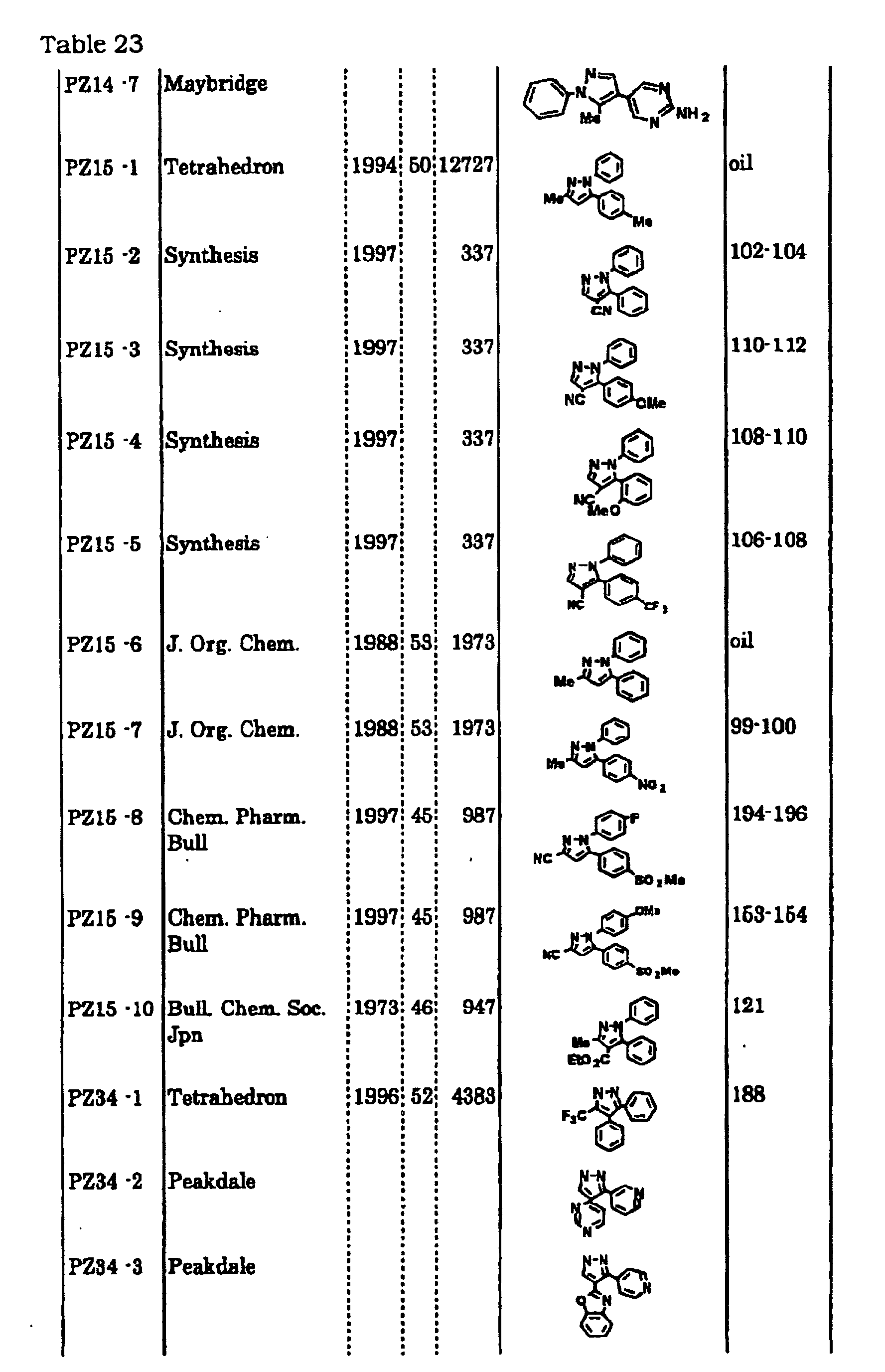

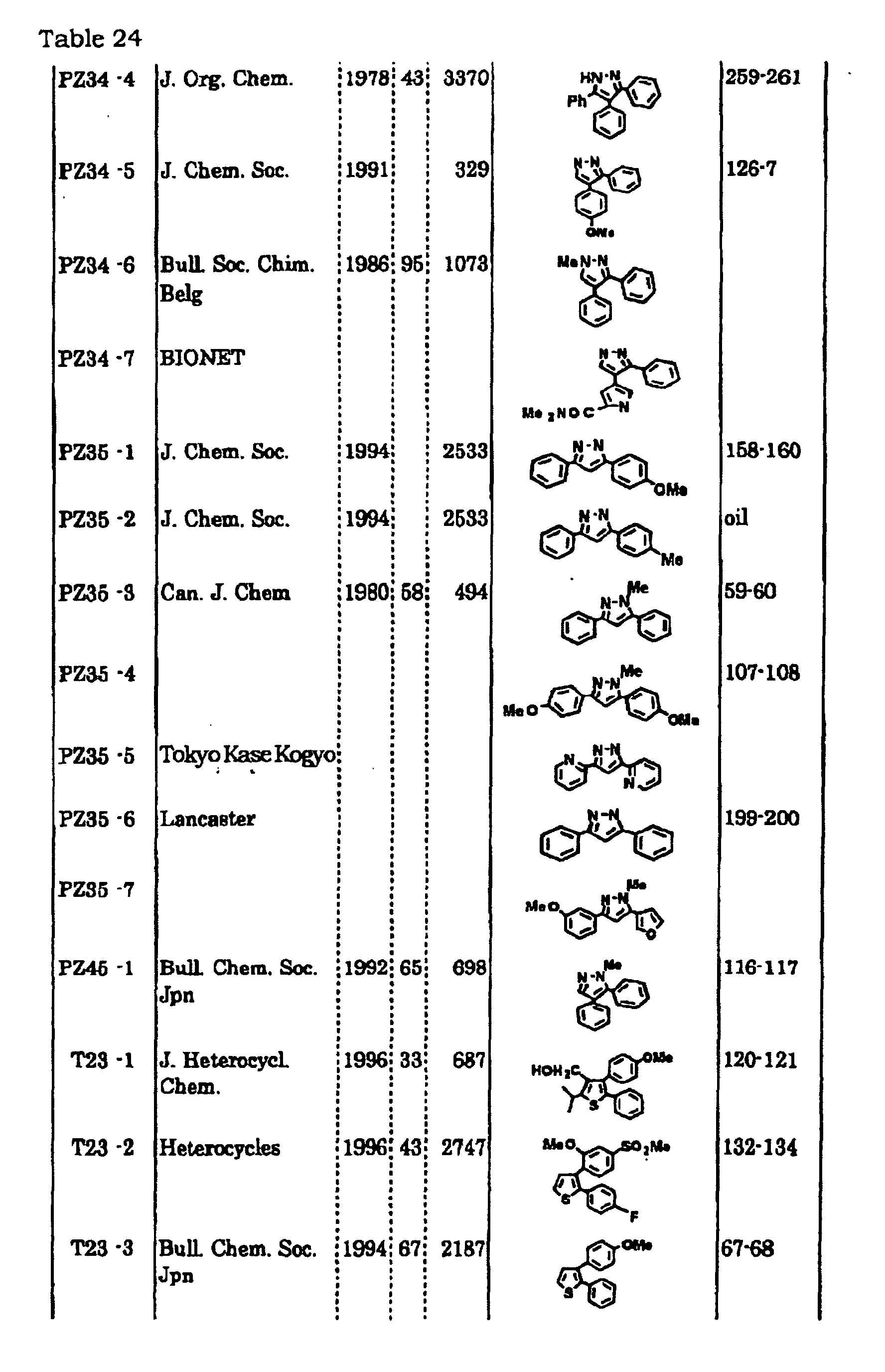

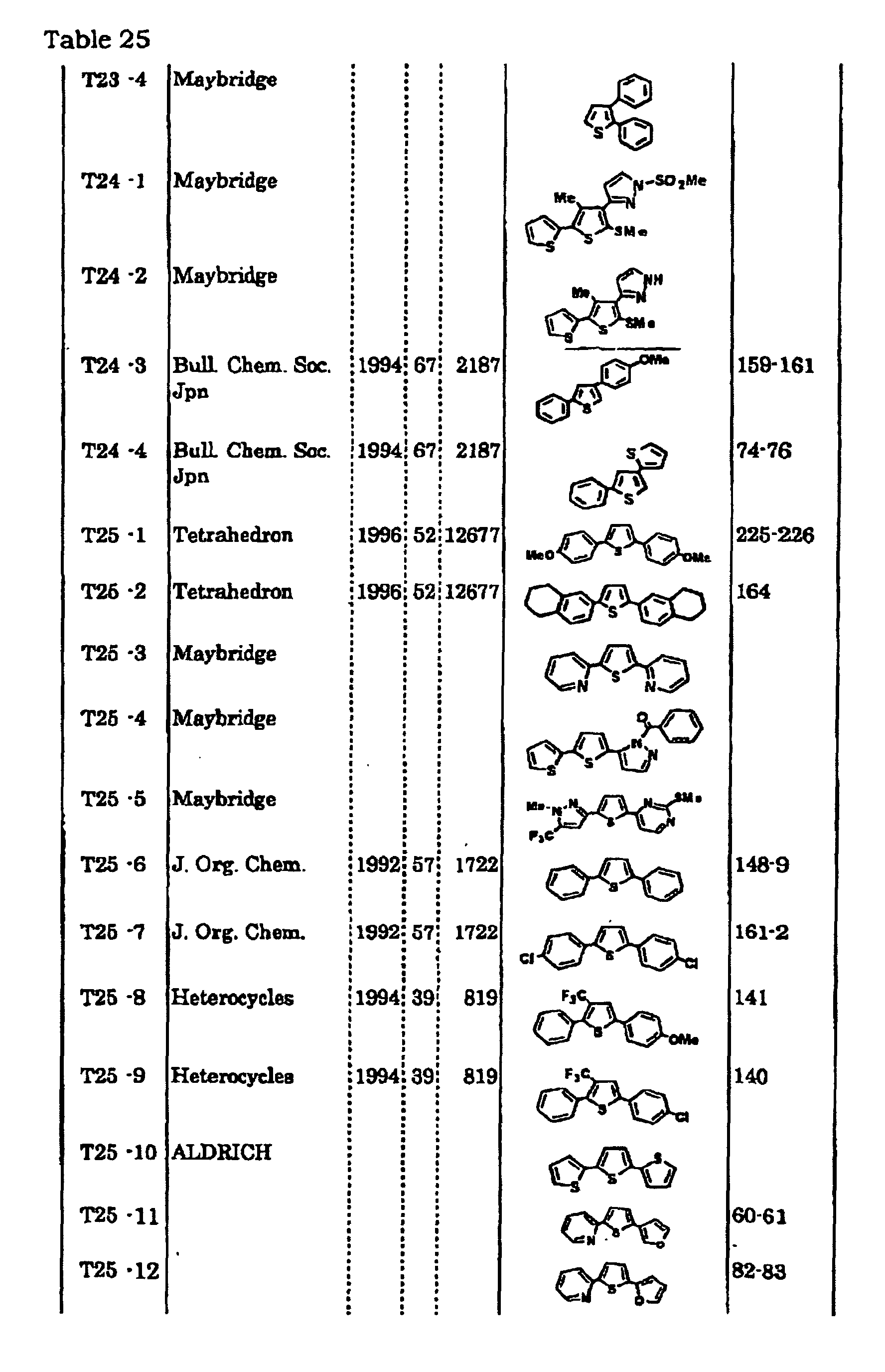

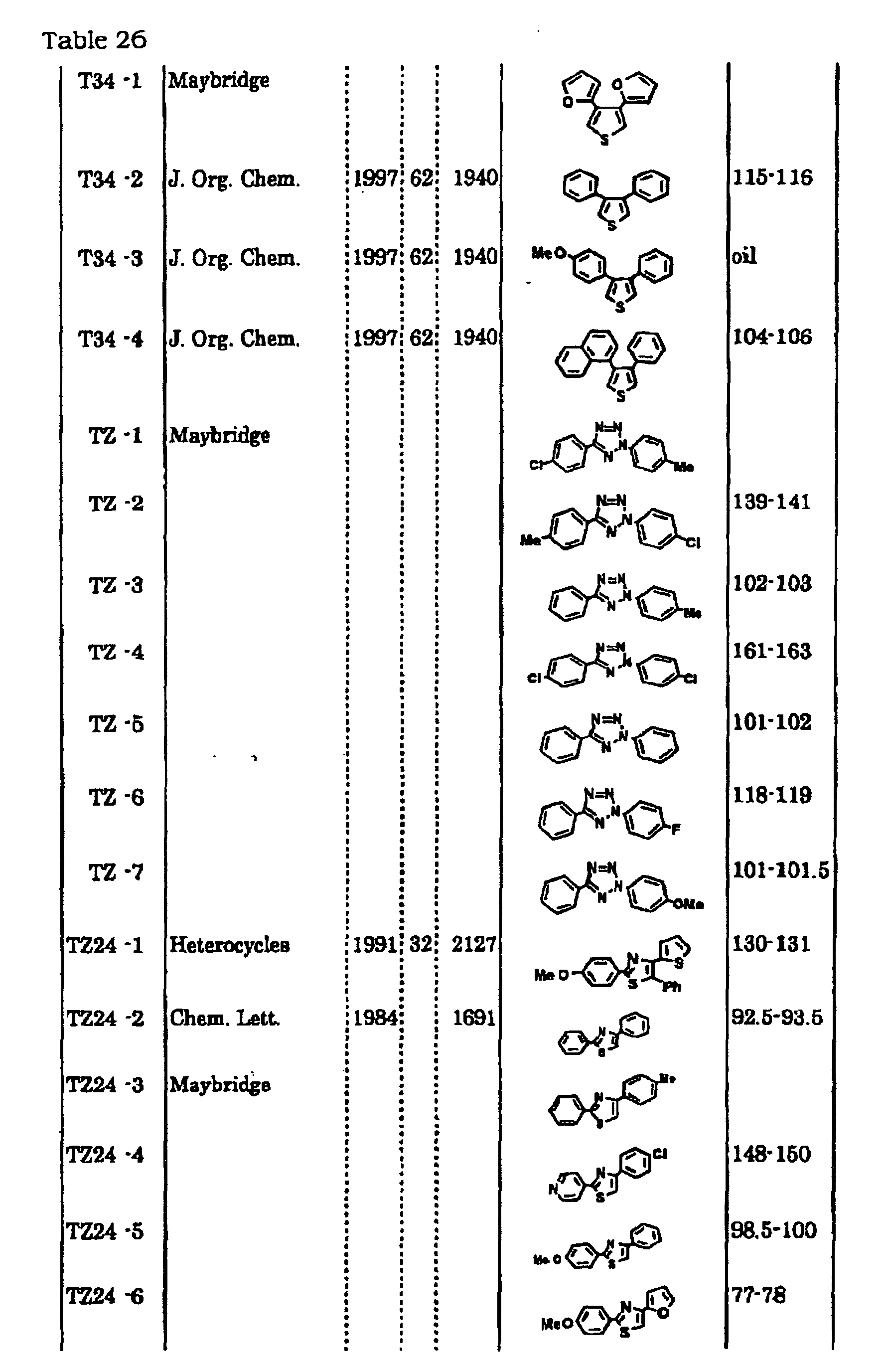

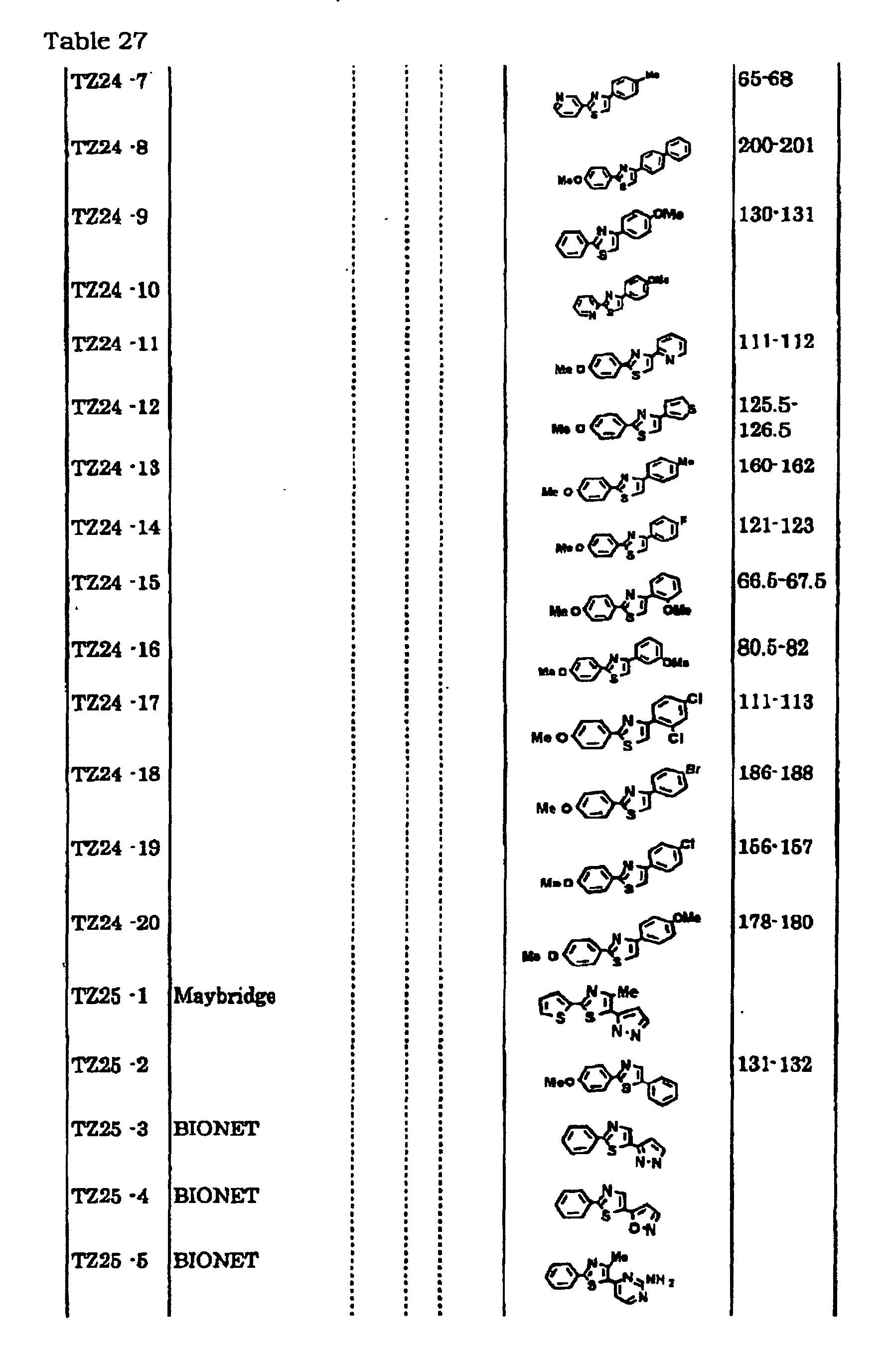

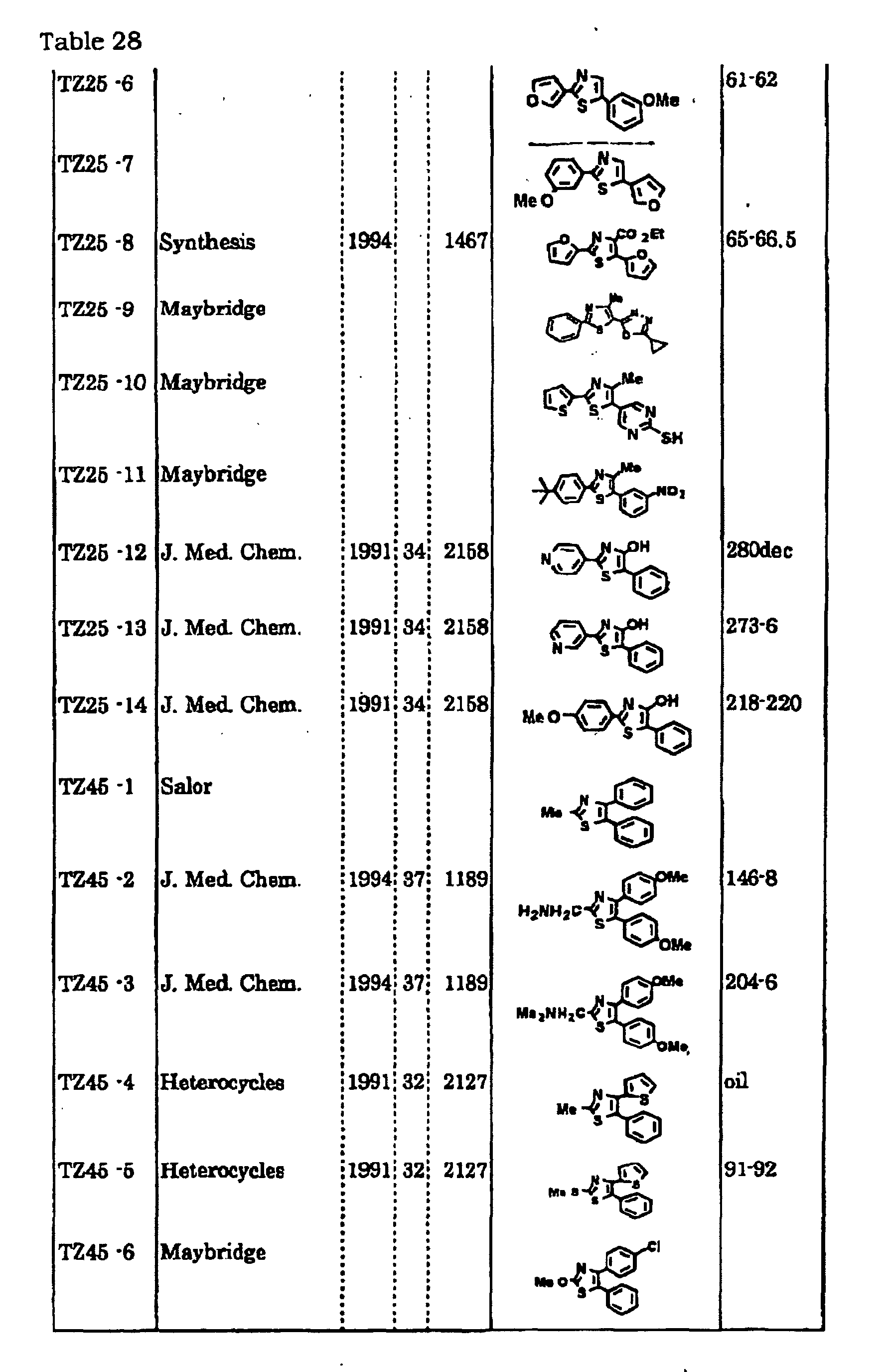

In the Tables, preferable compounds are 123TA14-2, 123TD45-6, 124OD35-12, 124OD35-13, 124OD35-14, 124OD35-15, 124TA35-17, 124TD35-6, 134OD25-9, 134OD25-10, 134OD25-11, 134OD25-12, 134OD25-13, 134OD25-14, 134OD25-15, 134OD25-16, 134OD25-17, 134OD25-18, 134OD25-19, 134OD25-20, 134OD25-21, 134OD25-22, 134OD25-23, 134OD25-24, 134OD25-25, 134OD25-26, 134OD25-27, 134OD25-28, 134OD25-29, 134OD25-30, 134OD25-31, 134OD25-32, 134OD25-33, 134OD25-34, 134OD25-35, 134OD25-36, 134OD25-37, 134OD25-38, 134OD25-39, 134OD25-40, 134OD25-41, 134OD25-42, 134OD25-43, 134OD25-44, 134OD25-45, 134OD25-46, 134OD25-47, 134OD25-48, 134OD25-49, 134OD25-50, 134TD25-2, 134TD25-3, 134TD25-4, 134TD25-5, 134TD25-6, F25-10, IM45-12, IM45-16, IX35-1, IX35-8, IX35-9, OX24-5, OX24-7, OX24-8, OX25-1, OX25-2, PZ35-4, PZ35-5, PZ35-6, T25-1, TZ-1, TZ-2, TZ-3 , TZ-4, TZ-5, TZ-6, TZ-7, TZ24-2, TZ24-3, TZ24-4, TZ24-5, TZ24-6, TZ24-7, TZ24-8, TZ24-9, TZ24-11 , TZ24-12, TZ24-13, TZ24-14, TZ24-15, TZ24-16, TZ24-17, TZ24-18, TZ24-19, TZ24-20, TZ25-2, and TZ25-6.

More preferable compounds are 123TA14-2, 124OD35-12, 124OD35-13, 124OD35-14, 124OD35-15, 124TA35-17, 124TD35-6, 134OD25-9, 134OD25-10, 134OD25-11, 134OD25-12, 134OD25-13, 134OD25-14, 134OD25-15, 134OD25-16, 134OD25-17, 134OD25-19, 134OD25-20, 134OD25-23, 134OD25-25, 134OD25-27, 134OD25-28, 134OD25-30, 134OD25-32, 134OD25-33, 134OD25-34, 134OD25-35, 134OD25-36, 134OD25-37, 134OD25-38, 134OD25-40, 134OD25-41, 134OD25-42, 134OD25-43, 134OD25-46, 134OD25-49, 134TD25-1, 134TD25-2, 134TD25-4, 134TD25-5, 134TD25-6, F25-10, IX35-1, IX35-12, IX35-13, IX35-8, IX35-9, OX24-5, OX24-7, OX24-8, OX25-1, OX25-2, PZ35-4, PZ35-5, PZ35-6, TZ-1, TZ-2, TZ-3, TZ-4, TZ-5, TZ-6, TZ-7, TZ24-2, TZ24-3, TZ24-5, TZ24-6, TZ24-7, TZ24-9, TZ24-11, TZ24-12, TZ24-13, TZ24-14, TZ24-16, TZ25-2, and TZ25-6.

Even more preferable compounds are 123TA14-2, 124OD35-12, 124OD35-15, 124OD35-13, 124TD35-6, 134OD25-9, 134OD25-10, 134OD25-11, 134OD25-15, 134OD25-14, 134OD25-23, 134OD25-28, 134OD25-27, 134OD25-32, 134OD25-40, 134OD25-46, 134TD25-1, 134TD25-4, 134TD25-5, F25-10, IX35-1, IX35-8, IX35-9, IX35-13, OX24-5, OX24-8, PZ35-4, PZ35-5, TZ-1, TZ-2, TZ-3, TZ-4, TZ-7, TZ24-3, TZ24-6, and TZ24-11.

### BEST MODE FOR CARRYING OUT THE INVENTION

The compound (I) according to the invention can be synthesized as follows. They can be synthesized by the method described in the literatures given in Tables 1 to 28 or are commercially available. Otherwise, they may be synthesized by the following processes.

### 1) Synthesis of pyrazole derivatives (PZ35)

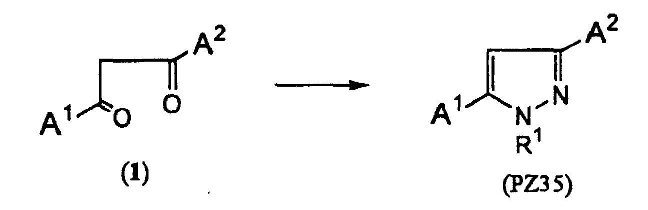

in which the symbols are as defined above.

Pyrazole derivative (PZ35) is prepared by heating 1,3-diketone (1) and hydrazine in a solvent. Alcohol may be used as a solvent, and the reaction may be conducted at a temperature between room temperature and a reflux temperature of the solvent.

### 2) Synthesis of oxazole derivatives (OX25)

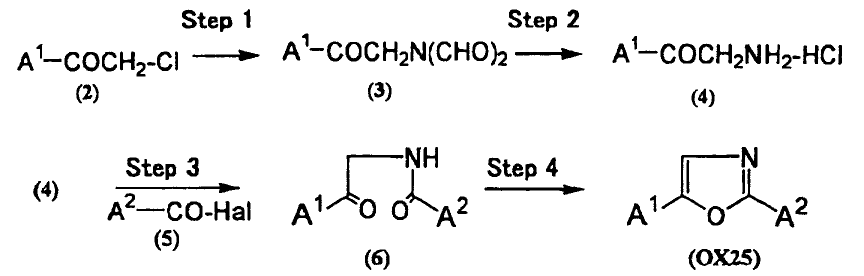

in which Hal is a halogen and the other symbols are as defined above.

Chloroacetophenone (2) is converted to aminoacetophenone (4) by the method of e.g. Synthesis, 112 (1990) or Tetrahedron Lett., 30, 5285 (1989). Compound (4) is acylated with acid halide and treated with phosphorus oxychloride, polyphosphoric acid, phosphorus trichloride, dimethyldichlorosilane, or the like in the absence or presence of a solvent, e.g., acetonitrile, dimethylformamide, toluene, or the like at a temperature between room temperature and reflux temperature of the solvent to give a cyclized product, oxazole (OX25).

### 3) Synthesis of thiazole derivatives (TZ24)

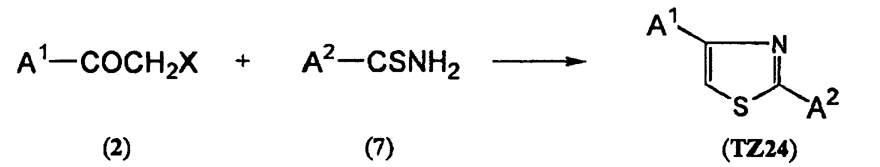

in which X is a halogen or toluenesulfonyloxy (hereinafter referred to OTs), and the other symbols are as defined above.

According to the method of e.g. J. Heterocycl. Chem., 28, 673 (1991), 2-halo-acetophenone (2) (e.g., 2-bromoacetophenone) is treated with thioamide (7) in a solvent e.g. alcohol, dimethylformamide, or the like, at a temperature between room temperature and reflux temperature of the solvent to give a thiazole derivative (TZ24) having A¹ and A² at positions 2 and 4, respectively.

Alternatively, acetophenone is converted to a corresponding tosylate (2: X=OTs) by the method of e.g. Synth. Commun., 28, 2371 (1998), which is then treated with thioamide (7) in a solvent e.g. dichloromethane, methanol, ethanol, or the like at a temperature between room temperature and reflux temperature of the solvent to give the same. 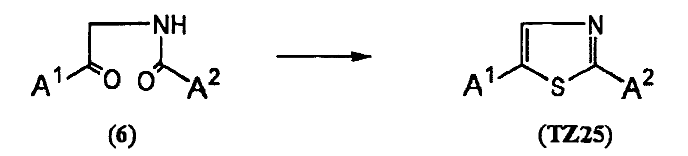 in which the symbols are as defined above.

According to the method of e.g. Collect. Czech. Chem, 58, 2720 (1993), ketoamide (6) is treated with Lawson reagent in a solvent e.g. benzene, toluene, xylene, dioxane, or the like at a temperature between room temperature and reflux temperature of the reaction solvent to give a thiazole derivative (TZ25) having A¹ and A² at positions 2 and 5, respectively.

### 4) Synthesis of 1,2,4-oxadiazole derivatives (124OD35)

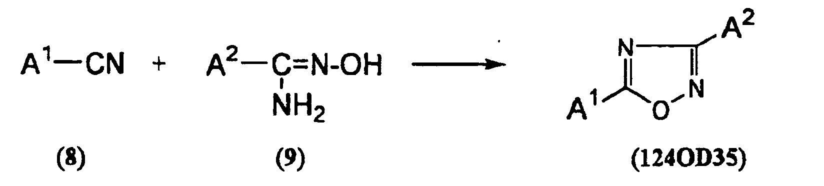

in which the symbols are as defined above.

According to the method of e.g. Tetrahedron 46, 3941 (1990), amidoxime (9) is treated with nitrile (8) in the presence of zinc chloride in a solvent e.g. ethyl acetate, butyl acetate, or the like at a temperature between room temperature and reflux temperature of the solvent to give 1,2,4-oxadiazole (124OD35).

### 5) Synthesis of 1,3,4-oxadiazole derivatives (134OD25)

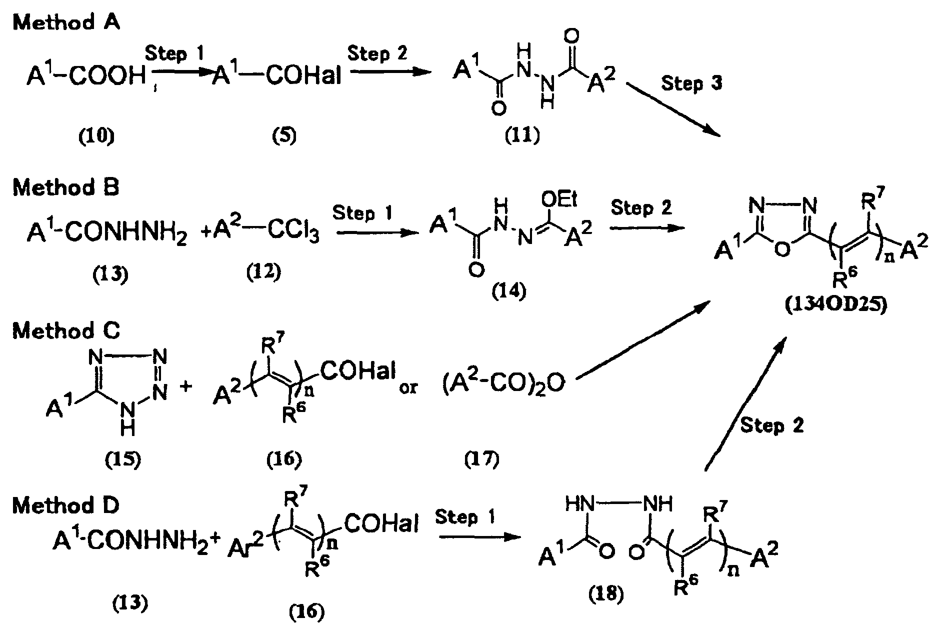

in which Hal is a halogen, n is 0 or 1, and the other symbols are as defined above.

### [Method A] (10 → 5 → 11 → 134OD25)

According to the method of, e.g. J. Org. Chem., 58, 2628 (1993), compound (134OD25) can be synthesized.
Step 1: When the starting material is carboxylic acid, it is converted into acid halide (5) using thionyl chloride, oxalyl chloride, or the like.
Step 2: A reaction of acid halide (5) and hydrazine monohydrate in the dichloromethane solvent at a temperature between ice cooling and reflux temperature of the solvent gives intermediate 1,2-bisbenzoylhydrazine (11).
Step 3: The intermediate (11) is cyclized with phosphorus oxychloride, polyphosphoric acid, phosphorus trichloride, dimethyldichlorosilane or the like in the absence or presence of a solvent, e.g., acetonitrile, dimethylformamide, toluene or the like at a temperature between room temperature and 150 °C to give 1,3,4-oxadiazole 134OD25.

### [Method B] (12 +□ 13 → 14 → 134OD25)

Method B follows the method of e.g. Synthesis, 946 (1979). In the presence of a base, phenyltrichloromethane (12) and hydrazide (13) are heated under reflux in alcohol solvent to give 134OD25.

In this reaction, the base may be sodium carbonate, pyridine, or the like, and the solvent may be alcohol, e.g. methanol, ethanol, or the like.

When the uncyclized intermediate (14) remains, it can be converted into 134OD25 e.g. by heating with an acid catalyst e.g. p-toluenesulfonic acid in a solvent e.g. dimethylformamide at 130 °C.

### [Method C] ( 15 +□ 16 or 17 → 134OD25 )

According to the method of e.g. J. Gen. Chem. USSR., 1125 (1992), tetrazole (15) and acid chloride (16) or acid anhydride (17) are heated at a temperature between 50 to 150 °C in the absence or presence of a solvent, e.g., acetonitrile, dimethylformamide, pyridine, toluene, or the like to synthesize 134OD25. The starting tetrazole (15) is commercially available or produced by the method of e.g. J.Org.Chem., 58 , 4139 (1993).

### [Method D] (13 + 16 → 18 → 134OD25 )

The intermediate (18) is obtained by the method of e.g. Khim Geterotsikl. Soedin., 333 (1996). The cyclization of (18) is carried out as in Step 3 of method A.

### 6) Synthesis of 1,2,4-triazole derivatives (124TA35)

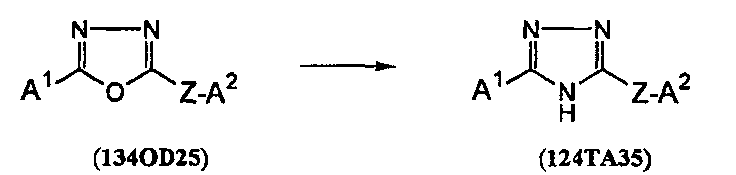

in which the symbols are as defined above.

In a sealed tube, 1,3,4-oxadiazole (134OD25) and thiourea in tetrahydrofuran solvent are heated at 100 to 150 °C to give 124TA35.

### 7) Synthesis of 1,3,4-thiadiazole derivatives134TD25

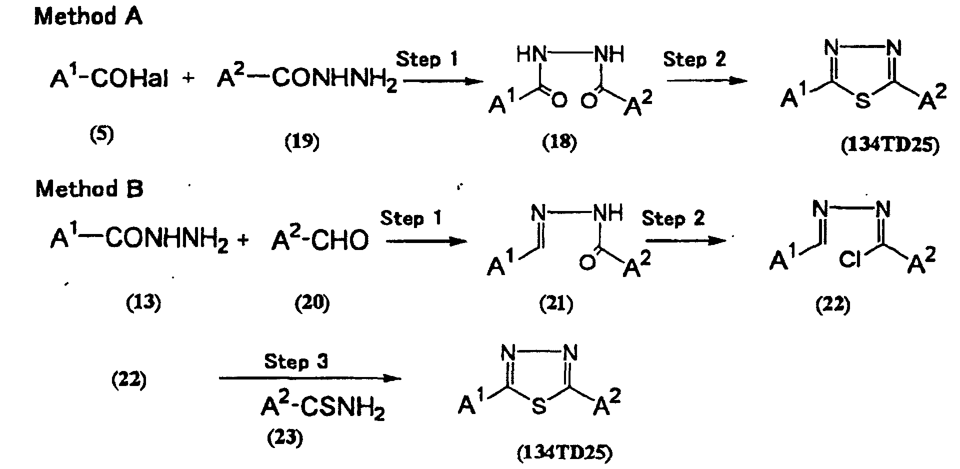

in which Hal is a halogen and the other symbols are as defined above.

### [Method A] ( 5 □+ 19 → 18 → 134TD25 )

The intermediate (18) is treated with phosphorus pentasulfide by the method of e.g. J. Prakt. Chem., 322, 933 (1980) to give 1,3,4-thiadiazole (134TD25).

### [Method B] ( 13 □+ 20 → 21 → 22 + 23 → 134TD25 )

According to the method of e.g. J. Chem. Soc. C, 1986 (1971) or J. Chem.Soc. Perkin Trans 1, 9, 1987 (1982), the intermediate (22) is prepared. This is then cyclized with thioamide (23) to give 1,3,4-thiadiazole (134TD25).

### 8) Synthesis of isoxazole derivatives (IX35)

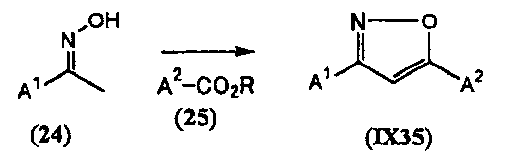

in which R is a lower alkyl and the other symbols are as defined above.

As described in e.g. Organic Synthesis Col. Vol. 6, 278 (1988), oxime (24) (prepared conventionally from the corresponding ketone) is treated with n-butyllitium in THF under ice cooling to form dianion. This is condensed with ester (25), followed by acid treatment to give isoxazole (IX35).

Pharmaceutical compositions of the invention (which enhance the expression of apoAI) activate a reverse cholesterol transport activity of HDL, an anti-inflammatory activity and an anti-coagulant activity, or the like. As a result, the compositions are useful for preventing and/or treating blood lipid disorders, arteriosclerotic diseases and coronary artery diseases caused by decreased level of HDL in plasma, as well as various cardiovascular diseases concomitant with them. "Blood lipid disorders" specifically include conditions of lowered level of serum HDL, hypercholesteremia, hypertriglyceridemia, or the like; "arteriosclerotic diseases" specifically include arteriosclerosis, or the like; "coronary artery diseases" specifically include myocardial infarction, ischaemic heart diseases, cardiac incompetence, or the like. "Various cardiovascular diseases concomitant with the above diseases" to be treated with the pharmaceutical compositions of the invention include hyperuricemia, corneal opacity, cerebrovascular disease, hereditary HDL deficiencies (Tangier disease, fish-eye disease), or the like.

The compositions of the invention may be administered orally or parenterally. For oral routes, the compositions may be formulated conventionally into usual dosage forms such as tablets, tablets, granules, powders, capsules, pills, solutions, syrups, buccals, sublinguals, or the like before administration. For parenteral administration, the compositions may be conventionally formulated into usual dosage forms such as injections, e.g., intramuscular or intravenous injections, suppositories; transdermal patches, inhalation, or the like.

A therapeutically effective amount of a compound according to the invention may be admixed with various suitable pharmaceutical additives such as excipient, binding agent, wetting agent, disintegrating agent, lubricant, diluent, or the like to give pharmaceutical compositions, if necessary. In the case of injections, the ingredients are sterilized together with a suitable carrier to formulate the composition.

More specifically, the excipients include lactose, sucrose, glucose, starch, calcium carbonate, crystalline cellulose, or the like; the binding agents include methyl cellulose, carboxymethylcellulose, hydroxypropylcellulose, gelatine, polyvinyl pyrrolidone, or the like; the disintegrating agents include carboxymethylcellulose, sodium carboxymethyl cellulose, starch, sodium alginate, algae powder, sodium lauryl sulfate, or the like; the lubricants include talc, magnesium stearate or Macrogol, or the like. Base materials of the suppository may be for example cacao butter, Macrogol, methylcellulose, or the like. Solutions, emulsions or suspensions for injection may comprise a solubilizing agent, a suspending agent, an emulsifying agent, a stabilizing agent, a preserving agent, an isotonic agent, or the like as usually used. Compositions for oral administration may comprise a flavoring agent, an aromatic agent, or the like.

Dose or therapeutically effective amount of the compounds according to the invention for enhancing the expression of apoAI is preferably determined considering age and body weight of patients, sort and severity of diseases to be treated, route of administration, or the like. In the case of oral administration to an adult, the dose range is usually 1 to 100 mg/kg/day, preferably 5 to 30 mg/kg/day. In the case of parenteral administration, the dose differs largely depending on the route of administration, but the dose range is usually 0.1 to 10 mg/kg/day, preferably 1 to 5 mg/kg/day. The dosage unit may be administered to a subject once or several times per day.

### EXAMPLES

Following references and examples are presented for purpose of further illustration of the invention, and they are not intended to limit the scope of the invention in any respect.

### Reference 1 2-Amino-3'-methoxyacetophenone hydrochloride (4-1)

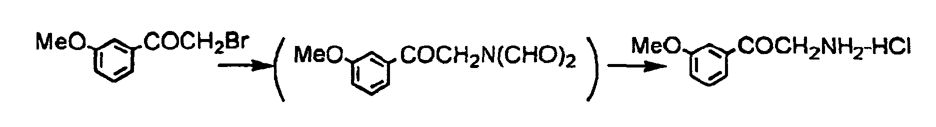

A suspension of 2-brom-3'-methoxyacetophenone (2.291 g, 10.00 mmol), and sodium diformylimide (1.102 g, 11.60 mmol ) in acetonitrile (5 mL) was stirred for 2 hours at room temperature and further stirred at 60 °C for 2 hours. Insoluble material in the reaction mixture was removed by filtration and the filtrate was concentrated *in vacuo*. Without purification, the residue was treated with 5 % hydrochloric acid - ethanol (25 mL), and the mixture was allowed to stand for 24 hours at room temperature. After evaporation of the solvent from the reaction mixture *in vacuo*, the resulting crystals were separated and washed successively with isopropyl ether and ethyl acetate to give crude crystals 4-1 (1.869 g, 92.7 %).
NMR (DMSO, d-6): 3.85 (3H, s), 4.59 (2H, s), 7.27-7.35 (1H, m), 7.45-7.56 (2H, m), 7.58-7.65 (1H, m), 8.42 (3H, br).

### Reference 2 3-Furoyl chloride (5-1)

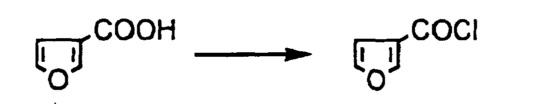

A mixture of furan-3-carboxylic acid (11.21g, 10.0 mmol ) and thionyl chloride (14.5 mL, 20.0 mmol) was stirred at 40 °C for 2 hours 30 minutes. The reaction product was purified by distillation under reduced pressure to give 3-furoyl chloride 5-1 (11.89g, 91.0 %) as colorless crystals (Caution: compound 5-1 is a potent irritant).
b.p. 68 - 72 °C (3325 Pa)

### Reference 3 N-(3'-Methoxyphenacyl)-3-furoylamide (6-1)

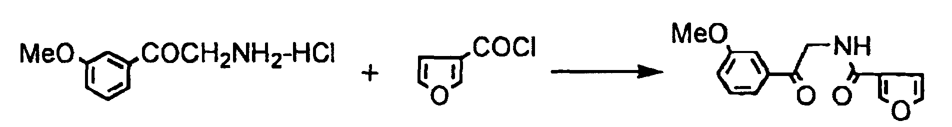

To a solution of compound 4-1 (1.008g, 5.00 mmol ) in pyridine (4 mL) was added 5-1 (0.685g, 5.25 mmol ) dropwise under ice cooling. The mixture was stirred at the same temperature for 3 hours and then at room temperature for 2 hours. After the solvent was evaporated *in vacuo*, ice and aqueous saturated sodium hydrogen carbonate were added and the mixture was extracted with ethyl acetate. The organic layer was washed with water and saturated brine, dried over anhydrous magnesium sulfate, and the solvent was evaporated *in vacuo*. The resulting crude crystals were recrystallized from ethyl acetate - hexane to give pale yellow prisms 6-1 (970 mg, 74.8 %).
m.p. 86-88 °C.
Elemental analysis: Calculated for C₁₄H₁₃NO₄-0.1H₂O: C, 64.41; H, 5.10; N, 5.37: Found: C, 64.50; H, 4.99; N, 5.45.
NMR (CDCl₃): 3.88 (3H, s), 4.90 (2H, d, J=4.2), 6.73 (1H, dd, J=0.9 and 2.1), 6.90 (1H, br), 7.15-7.22 (1H, m), 7.43 (1H, t, J=7.8), 7.48 (1H, t, J=1.8), 7.53 (1H, t, J=1.8), 7.61 (1H, d, J=7.5), 8.00-8.05 (1H, m).

### Reference 4 2-Furoyl-(3-methoxybenzylidene)hydrazide ( 21-1 )

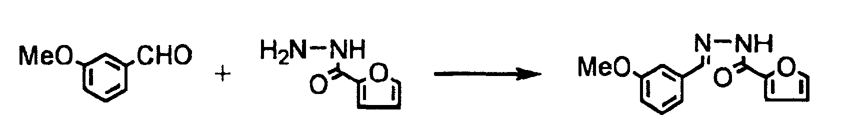

To a solution of 2-furoylhydrazide (2.522 g, 20.00 mmol) in ethanol (20 mL) was added dropwise *m*-anisaldehyde (2.43 mL, 19.97 mmol) at room temperature. After the mixture was stirred for 4 hours, it was allowed to stand overnight. The crystals precipitated from the reaction mixture were collected and washed with 95 % ethanol to give colorless prisms 21-1 (4.436 g, 90.8 %).
m.p. 156-157 °C.
Elemental analysis: Calculated for C₁₃H₁₂N₂O₃: C, 63.93; H, 4.95; N, 11.47: Found: C, 63.69; H, 4.98; N, 11.41.
NMR (CDCl₃): 3.87 (3H, s), 6.58 (1H, dd, J=1.5 and 3.3), 6.94-7.01 (1H, m), 7.24-7.44 (4H, m) 7.47-7.57 (1H, m), 8.24 (1H, s), 9.39 (1H, br).

### Reference 5 3-Acetamidobenzonitrile

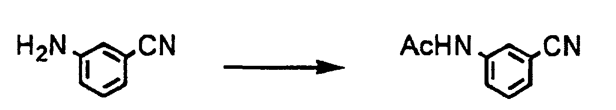

To 3-aminobenzonitrile (2.50, 21.16 mmol) was added acetic anhydride (10 mL, 105.98 mmol) at room temperature, and the mixture was stirred at the same temperature for 1 hour. The remaining reagent was evaporated *in vacuo*. To the residue was added saturated aqueous sodium hydrogen carbonate and the aqueous layer was extracted with ethyl acetate. The organic layer was washed with water, dried over anhydrous magnesium sulfate and the solvent was evaporated *in vacuo*. The residue was washed with isopropyl ether to give pale brown crystals (3.266 g, 96.3 %).
m.p. 120-123 °C.
Elemental analysis: Calculated for C₉H₈N₂O: C, 67.49; H, 5.03; N, 17.49: Found: C, 67.47; H, 5.01; N, 17.57.
NMR (CDCl₃): 2.21 (3H, s), 7.35-7.47 (3H, m), 7.67-7.75 (1H, m), 7.92 (1H, br).

### Reference 6. N-[3- (5-Tetrazolylphenyl)]-acetamide (15-1)

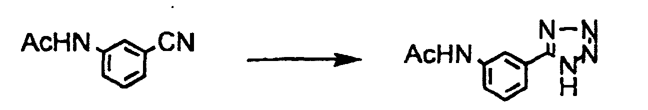

To a solution of 3-acetamidobenzonitrile (2.883 g, 18.00 mmol) in toluene (36 mL) were added trimethylsilyl azide (4.8 mL, 36.16 mmol) and di-n-butyltin oxide (0.448 g, 1.80 mmol), and the mixture was heated under reflux for 16 hours. The solvent was evaporated *in vacuo*, the residue was mixed with methanol, and the mixture was evaporated again *in vacuo*. The residue was extracted with saturated aqueous sodium hydrogen carbonate (1.81 g, 21.55 mmol) and the aqueous layer was washed with ethyl acetate. The alkaline aqueous solution was acidified with hydrochloric acid. The precipitated crystals were collected by filtration and washed with ethanol to give compound 15-1 (2.033 g, 55.6 %).
m.p. 250-260 °C (dec).
Elemental analysis: Calculated for C₉H₉N₅O: C, 53.20; H, 4.46; N, 34.46: Found: C, 53.25; H, 4.40; N, 33.52
NMR (DMSO, d-6): 2.09 (3H, s), 5.20 (1H, t, J=7.8), 7.62-7.78 (2H, m), 8.39 (1H, t, J=1.8), 10.20 (1H, s).

### Reference 7. 1,2-Bis(3-methylphenyl)hydrazine (11-1)

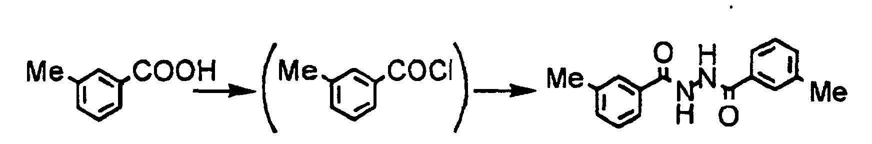

*m*-Toluic acid (10.89 g, 80.0 mmol) was treated with thionyl chloride (18.0 mL, 248.1 mmol) at 40 °C for 3 hours. Excess thionyl chloride was evaporated *in vacuo*. To a solution of crude *m*-toluic acid chloride in dry dichloromethane (44 mL) was added dropwise hydrazine monohydrate (11.5 mL, 237.08 mmol) at room temperature over 1 hour 30 minutes, and the mixture was stirred for 1 hour. The reaction mixture was added to water, and precipitated crystals were collected by filtration and washed with water and methanol to give colorless powdery crystals 11-1 (10.06g, 93.8 %).
m.p. 220-223 °C.
Elemental analysis: Calculated for C₁₆H₁₆N₂O₂: C, 71.62; H, 6.01; N, 10.44: Found: C, 71.27; H, 5.77; N, 10.61
NMR (DMSO, d-6): 2.39 (6H, s), 7.37-7.45 (4H, m), 7.68-7.78 (4H, m), 10.29 (2H, br)

### Reference 8 [(3-furoyl)-(3-methoxybenzoyl)]hydrazine (18-1)

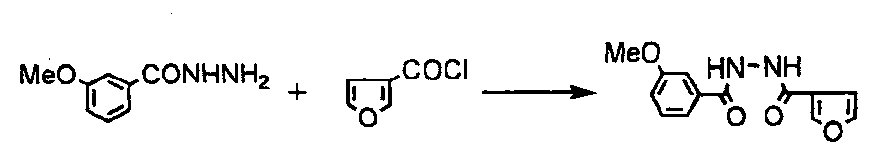

To a solution of *m*-anisic acid hydrazide (1.255 g, 7.552 mmol) in pyridine (4 mL) was added compound 5-1 (1.035g, 7.929 mmol) dropwise under ice cooling and the mixture was stirred at the same temperature for 4 hours, and then for 12 hours at room temperature. The solvent was evaporated *in vacuo* and precipitated crystals were washed with ethyl acetate and then isopropyl ether, and recrystallized from isopropanol to give colorless needles 18-1 (1.578 g, 80.3 %).
m.p. 211-212 °C.
Elemental analysis: Calculated for C₁₃H₁₂N₂O₄-0.5H₂O: C, 57.99; H, 4.87; N, 10.40: Found: C, 57.79; H, 4.83; N, 10.61.
NMR (DMSO, d-6): 3.82 (3H, s), 6.93 (1H, d, J=1.8), 7.11-7.23 (1H, m), 7.38-7.56 (3H, m), 7.80 (1H, d, J=1.8), 8.30 (1H, d, J=0.9), 10.23 (1H, br), 10.42 (1H, br).

### Reference 9 1-(3-Methoxyphenyl)ethanone oxime (24-1)

A mixture of 3-methoxyacetophenone (10 g), hydroxylamine hydrochloride (5.1 g), aqueous 4M-sodium hydroxide (18 mL), water (30 mL) and ethanol (50 mL) was heated at reflux for 2 hours. The solvent was removed *in vacuo* and resulting aqueous layer was extracted with ether. The organic layer was washed with water and brine and dried over anhydrous sodium sulfate. The solvent was evaporated *in vacuo*. Resulting oily substance was azeotropically dried two times with toluene and the residue was used in following steps without further purification.

### Example 1

### 3,5-Di(4-methoxyphenyl)-1-methylpyrazole (PZ35-4)

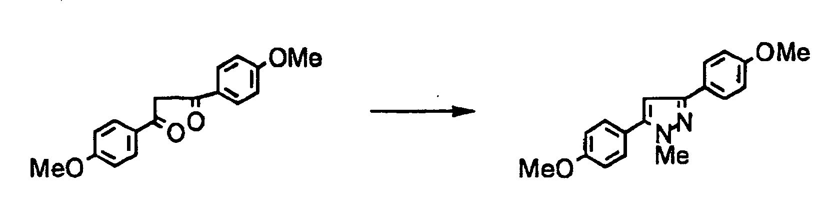

To a solution of 1,3-bis(4-methoxyphenyl)-1,3-propanedione (14.2 g, 5.0 mmol) in ethanol (10 mL) were added sodium hydrogen carbonate (1.68 g, 20.0 mmol) and methyl hydrazine sulfate (1.44 g, 10.0 mmol), and the mixture was heated at reflux for 3 hours. The solvent was evaporated *in vacuo* from the reaction mixture and residue was dissolved in chloroform. The organic layer was washed with water and brine, dried over anhydrous magnesium sulfate, and the solvent was evaporated *in vacuo*. The residue was recrystallized from methanol to give colorless prisms PZ35-4 (1.42 g, 96.6 %).
m.p. 107-108 °C.

### Example 2

### 2-(3-Furyl)-5-(3-methoxyphenyl)oxazole (OX25-2)

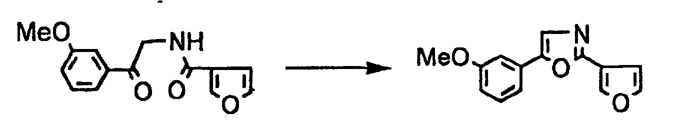

A suspension of compound 6-1 (778 mg, 3.00 mmol ) in phosphorus oxychloride (7.8 mL, 83.68 mmol) was stirred at 100 °C for 1 hour. Phosphorus oxychloride was removed *in vacuo*. The residue was mixed with ice, and the mixture was neutralized with aqueous concentrated ammonia, and extracted with ethyl acetate. The extracts were washed with water and brine, and dried over anhydrous magnesium sulfate, and the solvent was evaporated *in vacuo*. The residue was purified by silica gel chromatography (ethyl acetate - hexane = 1:3) followed by recrystallization from isopropyl ether to give pale yellow prisms OX25-2 (662 mg, 85.9 %).
m.p. 88-90 °C.

### Example 3

### 2-(4-Methoxyphenyl)-4-phenylthiazole (TZ24-5)

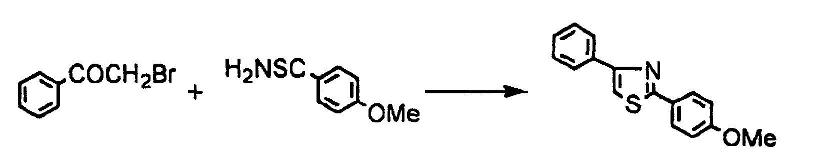

A suspension of α-bromoacetophenone (3.981 g, 20.00 mmol), 4-methoxythiobenzamide (3.345 g; 20.00 mmol) and dry ethanol (40 mL) was stirred at 50 °C for 2 hours. The solvent was evaporated *in vacuo*, the residue was mixed with ice, and the mixture was made weakly basic with 4N-sodium hydroxide and the aqueous layer was extracted with ethyl acetate. The organic layer was washed with water and saturated brine and dried over anhydrous magnesium sulfate. The solvent was evaporated *in vacuo*. The residue was purified by silica gel chromatography (chloroform), and recrystallization from ethyl acetate - hexane gave pale yellow prisms TZ24-5 (4.786 g, 89.5%).
m.p. 98.5-100 °C.

### Example 4

### 4- (2-Furyl)-2-(4-methoxyphenyl)thiazole (TZ24-6)

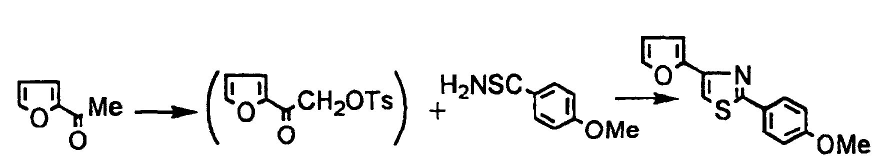

A suspension of 2-acetylfuran (0.661 g, 6.00 mmol) and hydroxy(tosyloxy)iodo-benzene (Koser's Reagent, 2.35 g, 6.00 mmol) in dry dichloromethane (12 mL) was stirred at room temperature for 16 hours. The solvent was evaporated *in vacuo*. To the residues were added 4-methoxythiobenzamide (1.00 g, 6.00 mmol) and dry ethanol (24 mL), and the mixture was heated at reflux for 4 hours. The solvent was evaporated *in vacuo*, water was added to the residue, and the mixture was extracted with ether. The ether layer was washed with water and brine, and was dried over anhydrous magnesium sulfate. The solvent was evaporated *in vacuo*. The residue was purified by silica gel chromatography (toluene) to give crude crystals, which were recrystallized from isopropyl ether - hexane giving TZ24-6 as pale brown crystals (668 mg, 43.4 %).
mp. 77 - 78 °C.

### Example 5

### 2- (3-Furyl)-5-(3-methoxyphenyl)thiazole (TZ25-6)

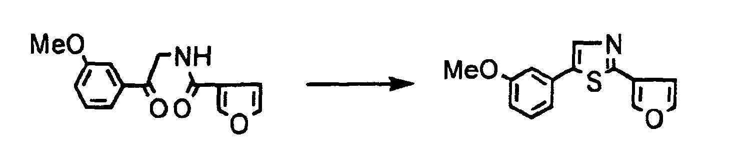

A suspension of compound 6-1 (1.063g, 4.00 mmol) and Lawson reagent (2. 10g, 5.19 mmol) in dry xylene (20 mL) was heated under reflux for 1 hour 30 minutes. The reaction mixture was mixed with aqueous saturated sodium hydrogen carbonate and extracted with ethyl acetate. The organic layer was washed with water and brine, dried over anhydrous magnesium sulfate, and the solvent was evaporated *in vacuo*. The residue was purified by chromatography on neutral alumina followed by silica gel eluted with ethyl acetate - hexane (1:4). Recrystallization from isopropyl ether gave TZ25-6 as pale brown prisms (681 mg, 66.1 %).
m.p. 61-62 °C.

### Example 6

### 5- (4-Methoxyphenyl)-3-phenyl-1,2,4-oxadiazole (124OD35-12)

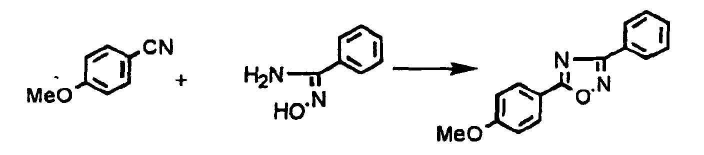

To a suspension of benzamidoxime (9.04 g, 66.40 mmol), zinc chloride (27.15 g, 199.22 mmol) and butyl acetate (68 mL) were added anisnitrile 8-1 (8.84 g, 66.39 mmol) and hydrogen chloride-ethyl acetate solution (4M, 17.1 mL, 68.40 mmol), and the mixture was heated under reflux at 130 °C for 3 hours. The reaction mixture was mixed with ice and the mixture was extracted with ethyl acetate. The extract was washed with water and brine, and dried over anhydrous magnesium sulfate. The solvent was evaporated *in vacuo*. The residue was subjected to silica gel chromatography and the fractions were eluted with ethyl acetate - hexane (1:9) to give 124OD35-12. This was recrystallized from isopropyl ether to give colorless prisms (3.779 g, 22.6 %).
m.p. 97-98 °C.

### Example 7

### 2,5-Bis (3-tolyl)-1,3,4-oxadiazole (134OD25-40)

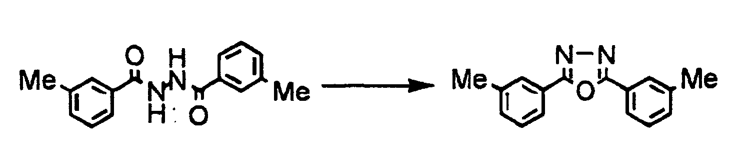

A mixture of compound 11-1 (5.37g, 20.01 mmol) and phosphorus oxychloride (18.7 mL, 200.6mmol) was stirred at 130 °C for 30 minutes. After evaporating phosphorus oxychloride *in vacuo*, the residue was mixed with ice, neutralized with aqueous ammonia, and the mixture was extracted with chloroform. The chloroform layer was washed with water and brine, and dried over anhydrous magnesium sulfate. The solvent was evaporated *in vacuo*. The residue was purified by silica gel chromatography (chloroform) and then recrystallized from ethyl acetate - hexane to give colorless prisms 134OD25-40 (2.996 g, 59.8 %).
m.p. 82-83 °C.

### Example 8

### 2- (2-Pyridyl)-5-phenyl-1,3,4-oxadiazole (134OD25-46)

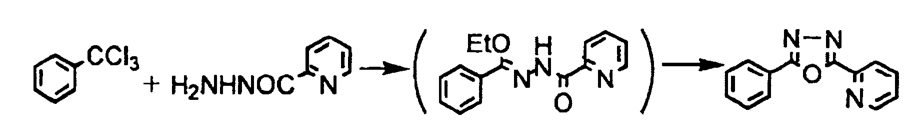

A suspension of phenyltrichloromethane (7.82 g, 40.00 mmol),α-picolininic acid hydrazide (5.48 g, 39.96 mmol) and sodium carbonate (4.02 g, 37.93 mmol) in dry ethanol (100 mL) was heated at reflux for 6 hours. After filtrating inorganic material off from the reaction mixture, the solvent was evaporated *in vacuo*. The residue was added to an aqueous saturated sodium hydrogen carbonate and the mixture was extracted with ethyl acetate. The ethyl acetate layer was washed with water and brine, dried over anhydrous magnesium sulfate, and the solvent was evaporated *in vacuo*.

The crude intermediate were stirred with p-toluenesulfonic acid hydrate (0.761g, 4.00 mmol) in dry dimethylformamide (20 mL) at 130 °C for 2 hours. The reaction mixture was added to an aqueous saturated sodium hydrogen carbonate and extracted with ethyl acetate. The organic layer was washed with water and brine, and dried over anhydrous magnesium sulfate. The solvent was evaporated *in vacuo*. The residue was purified by chromatography over neutral alumina and then silica gel (hexane - chloroform = 1:4).
Recrystallization from ethyl acetate - hexane gave 134OD25-46 as colorless prisms (3.036 g, 36.1 %).
m.p. 127-128 °C.

### Example 9

### 2- (4-Dimethylaminophenyl)-5-phenyl-1,3,4-oxadiazole (134OD25-15)

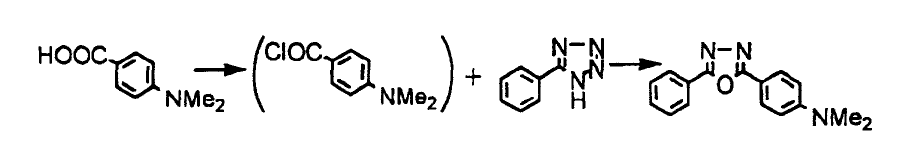

To a suspension of 4-dimethylaminophenylcarboxylic acid (1.652 g, 10.00 mmol), dry dimethylformamide (0.039 mL, 0.05 mmol) and dry dichloromethane (5 mL) was dropwise added oxalyl chloride (1.05 mL, 12.04 mmol) at room temperature over 10 minutes. The mixture was stirred for 1 hour, and the solvent was evaporated *in vacuo*. To the reaction product were added dry pyridine (0.81 mL, 10.01 mmol), dry acetonitrile (5 mL) and 5-phenyltetrazole (1.462 g, 10.00 mmol) and the mixture was heated under reflux for 2 hours 30 minutes. The reaction mixture was added to an aqueous saturated sodium hydrogen carbonate and the mixture was extracted with ethyl acetate. The organic layer was washed with water and brine and dried over anhydrous magnesium sulfate. The solvent was evaporated *in vacuo*. The residue was purified by silica gel chromatography eluting with ethyl acetate - chloroform (1: 15) and recrystallization from ethyl acetate - hexane gave 134OD25-15 as pale yellow prisms (422 mg, 15.9 %).
m.p. 135-140 °C.

### Example 10

### 2-[2-(2-Furyl)vinyl]-5-[1,3,4]-oxadiazole (134OD25-23)

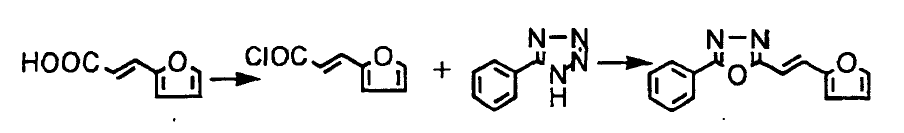

A suspension of 3-(2-furyl)acrylic acid (1.381 g, 10.00 mmol), thionyl chloride (0.80 mL, 11.03 mmol ), dimethylformamide (0.039 mL, 0.50 mmol) and acetonitrile (1.4 mL) was stirred at room temperature for 3 hours. The product was immediately mixed with 5-phenyltetrazole (1.462 g, 10.00 mmol), and the mixture was stirred at room temperature for 1 hour and at 100 °C for 3 hours. The reaction mixture was added to an aqueous saturated sodium hydrogen carbonate and extracted with ethyl acetate. The organic layer was washed with water and brine, dried over anhydrous magnesium sulfate, and the solvent was evaporated *in vacuo*. The residue was purified by silica gel chromatography (ethyl acetate - hexane = 1:3) and recrystallization from 95 % ethanol gave pale yellow prisms 134OD25-23 (653 mg, 27.4 %).
m.p. 131-132 °C.

### Example 11

### 2-(3-Furyl)-5-(3-methoxyphenyl)-[1,3,4]-oxadiazole (134OD25-32)

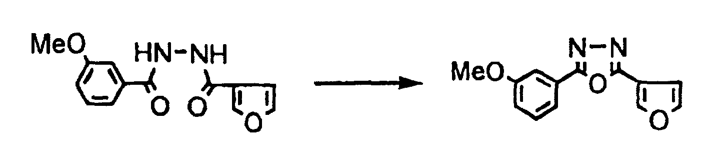

A suspension of compound 18-1 (5.04g, 19.37 mmol ) and phosphorus oxychloride (18.0 mL, 193.11m mmol) was stirred at 100 °C for 1 hour 30 minutes. After removal of phosphorus oxychloride *in vacuo*, the residue was added to ice, neutralized with aqueous concentrated ammonia, and extracted with ethyl acetate. The organic layer was washed with water and saturated brine, and dried over anhydrous magnesium sulfate, and the solvent was evaporated *in vacuo*. The residue was purified by silica gel chromatography and compound 134OD25-32 was obtained with ethyl acetate - hexane (1:3). The resulting crude crystals were recrystallized from isopropyl ether to give compound 134OD25-32 as colorless prisms (4.34g, 92.5 %).
m.p. 70-71 °C.

### Example 12

### 3-(3-Furyl)-5-(3-methoxyphenyl)-[1,2,4]-triazole (124TA35-17)

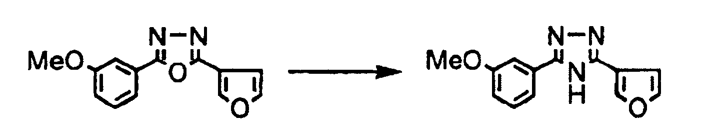

A suspension of compound 134OD25-32 (1.211 mg, 5.00 mmol) and thiourea (1.00 g, 13.14 mmol) in tetrahydrofuran (5 mL) was heated at 150 °C for 24 hours in a sealed tube. The reaction mixture was added to an aqueous saturated sodium hydrogen carbonate and extracted with ethyl acetate. The extract was washed with water and brine, dried over anhydrous magnesium sulfate, and the solvent was evaporated *in vacuo*. The residue was purified by silica gel chromatography. Elution with ethyl acetate - hexane (1:1) followed by recrystallization from ethyl acetate gave 124TA35-17 (471 mg, 39.1 %).
m.p. 169-171 °C.

### Example 13

### 2-(3-Furyl)-5-(3-methoxyphenyl)-[1,3,4]-thiadiazole (134TD25-2)

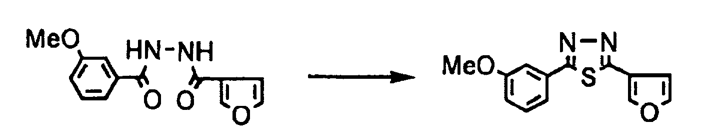

A suspension of compound 18-1 (1.562g, 6.00 mmol) and phosphorus pentasulfide (1.80 g, 8.10 mmol) in dry pyridine (12 mL) was stirred at 100 °C for 9 hours. After the solvent was removed *in vacuo*, the residue was mixed with ice, made weakly alkaline with 4M-sodium hydroxide, and extracted with ethyl acetate. The organic layer was washed with water and brine, and dried over anhydrous magnesium sulfate, and the solvent was evaporated *in vacuo*. The residue was purified by silica gel chromatography and eluted with ethyl acetate. The eluent (2.529g) still contained an uncyclized intermediate, which was mixed with p-toluenesulfonic acid hydrate (0.395 g, 2.08 mmol) and dry toluene (25 mL), and the mixture was heated under reflux for 30 minutes. The reaction mixture was added to an aqueous saturated sodium hydrogen carbonate and extracted with ethyl acetate. The ethyl acetate layer was washed with water and brine, dried over anhydrous magnesium sulfate and the solvent was evaporated *in vacuo*. The residue was purified by silica gel chromatography (ethyl acetate - hexane = 1:3) and recrystallization from 95 % ethanol gave compound 134TD25-2 (0.820g, 52.9 %).
m.p. 75.5-76.5 °C.

### Example 14

### 2-(2-Furyl)-5-p-tolyl-[1,3,4]-thiadiazole (134TD25-5)

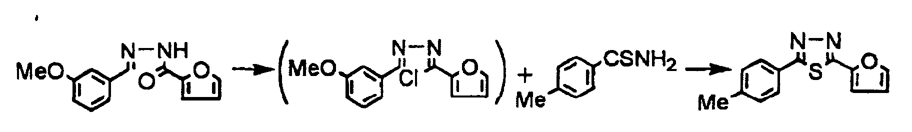

A suspension of compound 21-1 (2.931g, 12.00 mmol) and thionyl chloride (1.04 mL, 14.34 mmol) in benzene (12 mL) was heated under reflux for 6 hours. The solvent was evaporated *in vacuo*, and the residue was treated with hot petroleum ether. Only the soluble portion was taken up and the solvent was evaporated *in vacuo*. The resulting crude product (1.521 g) and 4-methylthiobenzamide (0.875 g, 5.786 mmol) were dissolved in dry ethanol (20 mL) and the solution was stirred at room temperature for 30 hours. The solvent was removed *in vacuo* from reaction mixture. The residue was purified by silica gel chromatography (ethyl acetate - hexane = 1:5) and recrystallization from 95 % ethanol gave compound 134TD25-5 (670 mg, 23.0 %).
m.p. 111-113 °C.

### Example 15

### 5-(Furan-3-yl)-3-(3-methoxyphenyl)-isoxazole (IX35-9)

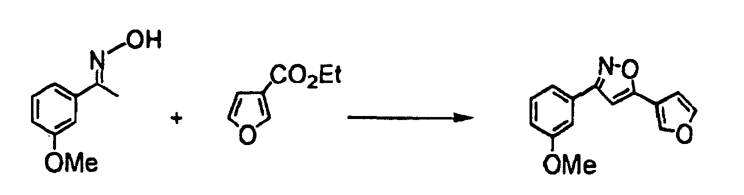

To a solution of 1-(3-methoxyphenyl)ethanone oxime (1.65 g, 0.01 mol) in THF (55 mL) was added dropwise a solution of n-butyllithium (1.6M-in hexane, 14 mL) under ice cooling. After stirring at the same temperature for 30 minutes, a solution of ethyl furan-3-carboxylate (0.7g, 5 mmol) in THF (10 mL) was added slowly. After stirring for 1 hour at ice bath temperature, 5N-hydrochloric acid (18 mL) was added in one portion, and the mixture was heated at reflux for 1 hour. After cooling, the reaction mixture was poured into ice, made alkaline with sodium hydrogen carbonate, and extracted with ether. The reaction product was purified by silica gel chromatography (82 g, ethyl acetate - hexane = 1:4) to give a mixture mainly containing compound IX35-9. This mixture was again purified by silica gel chromatography (90g, toluene) to give colorless crystals (480 mg), which were further recrystallized from acetone - hexane to obtain IX35-9 as colorless crystals.
m.p. 36-37 °C.

Other compounds (I) were synthesized in a similar manner. Their physiological constants are listed below:

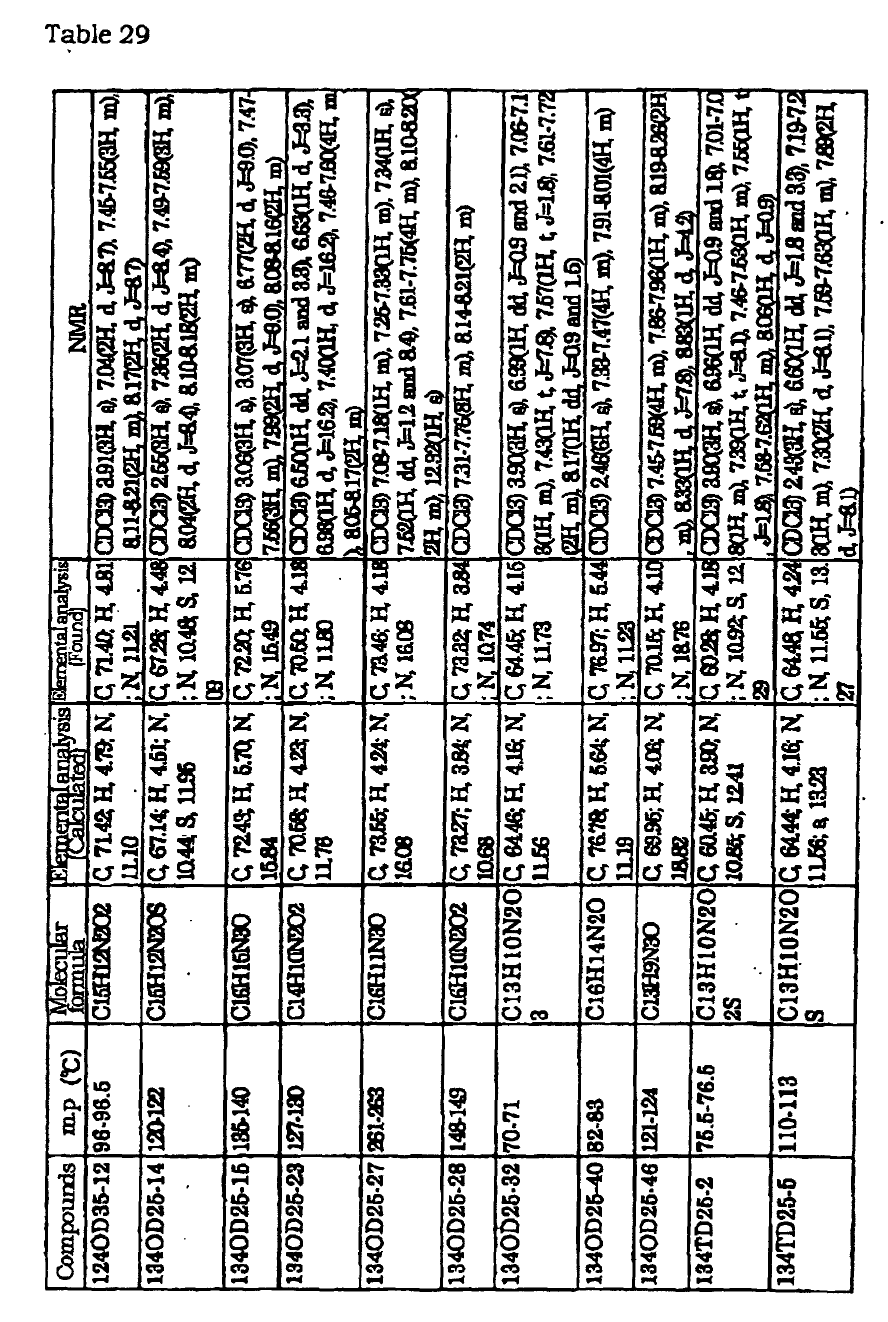

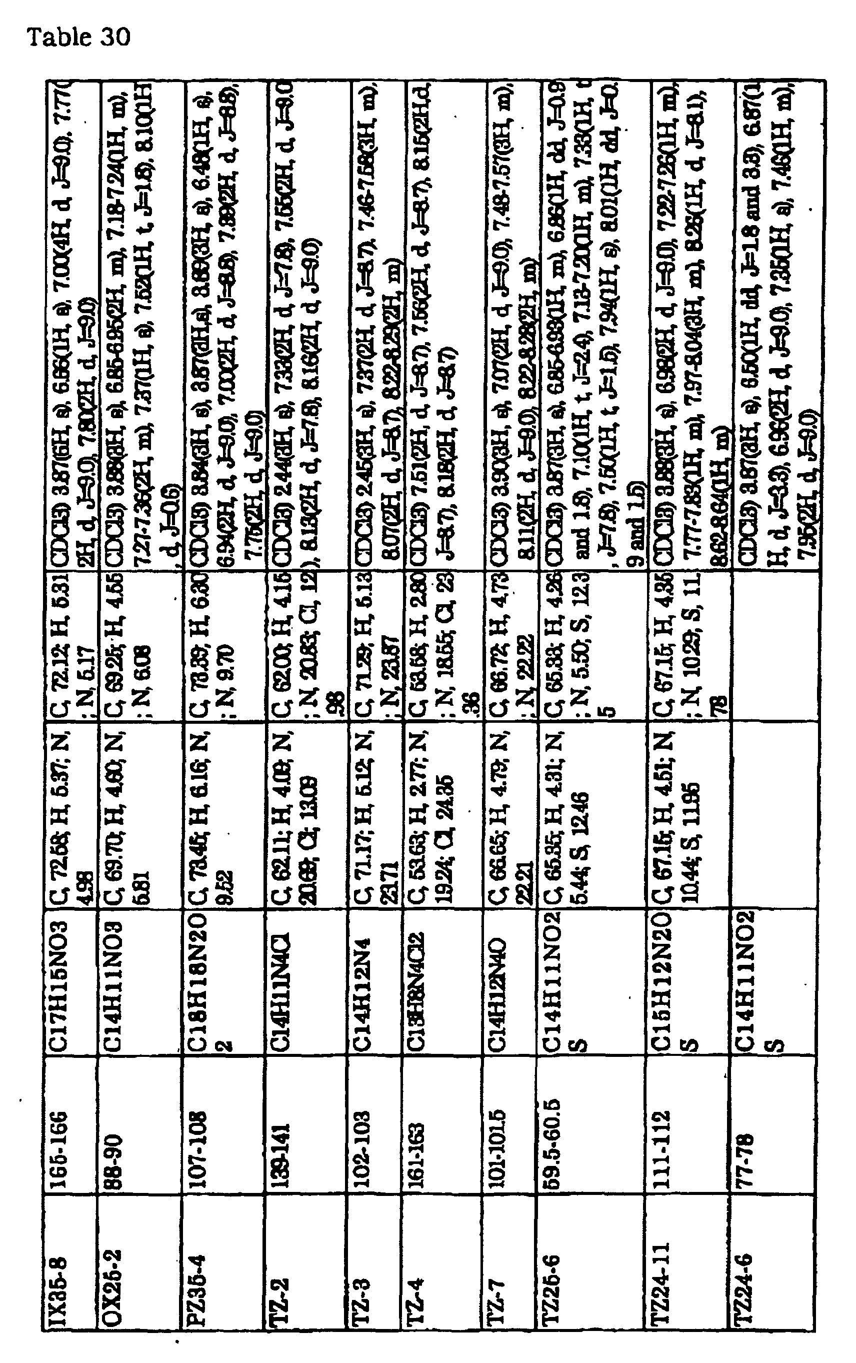

### Experiment 1

### Activity to enhance the production of human apoAI

The promoter region of the gene encoding human apoAI was isolated and ligated upstream the structure gene of firefly luciferase to construct a reporter plasmid. The reporter plasmid and a marker plasmid conferring the neomycin resistance were co-infected to cell lines derived from human hepatoma, HepG2 cells, and the cell lines were incubated in a selection medium comprising DMEM medium containing 10 % fetal calf serum supplemented with G418 (Final concentration: 0.7 mg/mL, Gibco) to give established strains that stably express the reporter molecule. The strains were seeded to a 96-well culture plates at a density of 50,000 cells per well, and incubated for 48 hours at 37 °C under 5% carbon dioxide. Then, a solution of the compounds according to the invention in DMSO was added to the wells at a final concentration of 0 to 10 µg/mL. After further incubation for 24 hours, the cells were added with a luciferase assay reagent (Piccagene LT 7.5 registered trade mark, Toyo Ink, KK), and the luciferase activity was determined using a luminometer (MicroBetaTM TRILUX, 1 sec/well, Wallac). The concentration of the compounds, which intensified the luciferase activity twice compared to that of control (DMSO without any compound of the invention added) was set as the minimal effective dose (MED). The results are shown in Table 31.

**Table 31**

| Compound | MED (µM) |
|---|---|
| 123TA14-2 | 0.59 |
| 124OD35-12 | 0.07 |
| 124OD35-15 | 0.18 |
| 124OD35-13 | 0.7 |
| 124TD35-6 | 0.93 |
| 134OD25-9 | 0.22 |
| 134OD25-10 | 0.91 |
| 134OD25-11 | 0.74 |
| 134OD25-15 | 0.27 |
| 134OD25-14 | 0.56 |
| 134OD25-23 | 0.82 |
| 134OD25-28 | 1.1 |
| 134OD25-27 | 2.4 |
| 134OD25-32 | 2.8 |
| 134OD25-34 | 1.5 |
| 134OD25-40 | 0.17 |
| 134OD25-46 | 0.37 |
| 134TD25-1 | 0.89 |
| 134TD25-4 | 0.58 |
| 134TD25-5 | 0.98 |
| F25-10 | 2.5 |
| IX35-1 | 0.75 |
| IX35-8 | 0.5 |
| IX35-9 | 0.53 |
| OX24-5 | 0.32 |
| OX24-8 | 2.9 |
| PZ35-4 | 0.41 |
| PZ35-5 | 1.5 |
| TZ-1 | 0.33 |
| TZ-2 | 0.42 |
| TZ-3 | 0.2 |
| TZ-4 | 0.53 |
| TZ-7 | 0.22 |
| TZ24-3 | 0.45 |
| TZ24-5 | 3.7 |
| TZ24-6 | 1.2 |
| TZ24-11 | 1.2 |

Table 31 shows that the compounds according to the invention can promote the function of the gene encoding human apoAI, thus indicating enhancement of the expression of apoAI.

| Formulation 1 tablets | |
|---|---|
| compound (134OD25-32) | 15 mg |
| starch | 15 mg |
| lactose | 15 mg |
| crystalline cellulose | 19 mg |
| polyvinyl alcohol | 3 mg |
| distilled water | 30 mL |
| calcium stearate | 3 mg |

The ingredients other than calcium stearate were mixed uniformly, powdered, granulated, and dried to give granules of a suitable size. Then the calcium stearate was added and the materials were compressed to give a tablet formulation.

| Formulation 2 Capsules | |
|---|---|
| compound (134OD25-40) | 10 mg |
| magnesium stearate | 10 mg |
| lactose | 80 mg |

The ingredients were homogeneously mixed to give powder or fine particles to give a powder formulation. This was filled in capsules to give a capsule formulation.

| Formulation 3 Granules | |
|---|---|
| compound (124OD35-12) | 30 g |
| lactose | 265 g |
| magnesium stearate | 5 g |

The ingredients were mixed thoroughly, compressed, powdered, granulated and sieved to give a granule formulation.

### INDUSTRIAL APPLICABILITY

As is apparent from the experiment as described above, the compounds according to the invention have an activity for enhancing the expression of apoAI. Thus, the compounds according to the invention are very useful as pharmaceutical compositions for preventing and/or treating blood lipid disorders, arteriosclerotic diseases, or coronary artery diseases.

## Claims

1. A pharmaceutical composition for enhancing the expression of apoAI, which comprises a compound of formula (I): 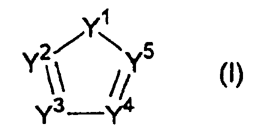 in which
Y¹ is O, S or NR¹;
Y² is CR² or N;
Y³ is CR³ or N;
Y⁴ is CR⁴ or N;
Y⁵ is CR⁵ or N;
R¹ is A¹, -Z-A², a hydrogen, a lower alkyl that may be optionally substituted, an acyl that may be optionally substituted, an amino that may be optionally substituted, a lower alkoxycarbonyl that may be optionally substituted, or a carbamoyl that may be optionally substituted;
R², R³, R⁴ and R⁵ are independently A¹, -Z-A², a hydrogen, a halogen, a hydroxy, a lower alkyl that may be optionally substituted, a lower alkoxy that may be optionally substituted, a nitro, an acyl that may be optionally substituted, an amino that may be optionally substituted, a mercapto, a lower alkylthio that may be optionally substituted, a carboxy, a lower alkoxycarbonyl that may be optionally substituted, or a carbamoyl that may be optionally substituted;
A¹ and A² are independently a cycloalkyl that may be optionally substituted, an aryl that may be optionally substituted, or a heterocyclic ring that may be optionally substituted;
-Z- is a single bond, -CR⁶=CR⁷-, or -N-, wherein R⁶ and R⁷ are independently a hydrogen or a lower alkyl;
provided that at least one selected from Y¹, Y², Y³, Y⁴, and Y⁵ has A¹, and any one of the others has -Z-A²; a prodrug thereof, a pharmaceutically acceptable salt or solvate of them.

2. The pharmaceutical composition according to claim 1, in which the 5-membered ring consisting of Y¹, Y², Y³, Y⁴, and Y⁵ has a nucleus selected from a group consisting of 1,2,3-triazole, 1,2,4-triazole, 1,2,3-thiadiazole, 1,2,4-thiadiazole, 1,2,5-thiadiazole, 1,3,4-thiadiazole, 1,2,3-oxadiazole, 1,2,4-oxadiazole, 1,2,5-oxadiazole, 1,3,4-oxadiazole, pyrazole, tetrazole, oxazole, isoxazole, thiazole, isothiazole, pyrrole, furan and thiophene.

3. The pharmaceutical composition according to claim 2, in which the 5-membered ring consisting of Y¹, Y², Y³, Y⁴, and Y⁵ has a nucleus selected from a group consisting of 1,2,3-triazole, 1,2,4-triazole, 1,2,4-thiadiazole, 1,3,4-thiadiazole, 1,2,4-oxadiazole, 1,3,4-oxadiazole, pyrazole, tetrazole, oxazole, isoxazole, thiazole, furan, and thiophene.

4. The pharmaceutical composition according to any one of claims 1 to 3, in which A¹ and A² are independently a phenyl, a pyridyl, a pyrazinyl, a furyl, a thienyl, a thiazolyl, a pyrazolyl, a isoxazolyl, a benzofuryl, or an indolyl, each of which may be optionally substituted.

5. The pharmaceutical composition according to claim 4, in which A¹ and A² are independently a phenyl that may be optionally substituted by a halogen, a hydroxy, a lower alkyl, a lower alkoxy, a lower alkylthio, an amino that may be optionally substituted by a lower alkyl, a phenyl, a styryl or a heteroaryl; a thiazolyl that may be optionally substituted by a lower alkyl; a pyrazolyl that may be optionally substituted by a lower alkyl; an unsubstituted pyridyl; an unsubstituted indolyl; an unsubstituted benzofuryl; an unsubstituted thienyl; or an unsubstituted furyl.

6. The pharmaceutical composition according to any one of claims 1 to 5, in which Z is a single bond.

7. The pharmaceutical composition according to any one of claims 1 to 6, in which Y¹ is O, S or NR¹, R¹ is a lower alkyl that may be optionally substituted, or an amino that may be optionally substituted; and, among Y², Y³, Y⁴ and Y⁵, one or two is (are) independently CA¹, one is CA², and the others are independently CH or N.

8. The pharmaceutical composition according to any one of claims 1 to 7, which is used for prevention and/ or treatment of blood lipid disorders, arteriosclerotic diseases or coronary artery diseases.

9. A method of enhancing the expression of apoAI, which comprises administrating a therapeutically effective amount of a compound of formula (I) as defined in claim 1, a prodrug thereof, a pharmaceutically acceptable salt or solvate of them to a patient expected to enhance the expression of apoAI.

10. A method of treatment and/or prevention of blood lipid disorders, arteriosclerotic diseases or coronary artery diseases, which comprises administrating a therapeutically effective amount of a compound of formula (I) as defined in claim 1, a prodrug thereof, a pharmaceutically acceptable salt or solvate of them to a patient suspected to have blood lipid disorders, arteriosclerotic diseases or coronary artery diseases.

11. Use of a compound of formula (I) as defined in claim 1, a prodrug thereof, a pharmaceutically acceptable salt or solvate of them for the manufacturing a medicament of enhancing the expression of apoAI.

12. Use of a compound of formula (I) as defined in claim 1, a prodrug thereof, a pharmaceutically acceptable salt or solvate of them for the manufacturing a medicament of treatment and/or prevention of blood lipid disorders, arteriosclerotic diseases or coronary artery diseases.
